# EUROPEAN PATENT APPLICATION

(11) **EP 4 509 142 A1**
(43) Date of publication of application: **19.02.2025**
(21) Application number: 23382852.4
(22) Date of filing: 16.08.2023
(51) Int. Cl.: A61K 47/68, A61P 35/00, C07K 16/28, A61K 31/00

(54) **FGFR4 AS TARGET IN CANCER TREATMENT**

(71) Applicant: Ona Therapeutics S.L., 08028 Barcelona (ES); Fundació Institut de Recerca Biomèdica (IRB Barcelona), 08028 Barcelona (ES); Institució Catalana de Recerca i Estudis Avançats, 08010 Barcelona (ES); Hospital Clínic de Barcelona, 08036 Barcelona (ES); Fundació de Recerca Clínic Barcelona-Institut d'Investigacions Biomèdiques August Pi I Sunyer, 08036 Barcelona (ES); Universitat de Barcelona, 08028 Barcelona (ES)
(72) Inventor: VANHOOREN, Valerie Liesbeth Brigitte, 08028 Barcelona (ES); DE FRIAS SÁNCHEZ, Mercè, 08028 Barcelona (ES); SUN, Haijun, 08028 Barcelona (ES); MORANCHO ARMISEN, Beatriz, 08028 Barcelona (ES); GUARDIOLA BAGÁN, Salvador, 08028 Barcelona (ES); GONZÁLEZ GIRONÈS, Diana María, 08028 Barcelona (ES); GOMIS CABRÉ, Roger, 08010 Barcelona (ES); GREGORIO JORDAN, Sara, 08028 Barcelona (ES); BLASCO LÁZARO, Teresa, 08028 Barcelona (ES); PRAT APARICIO, Aleix, 08036 Barcelona (ES); BRASÓ MARISTANY, Fara, 08036 Barcelona (ES)
(74) Representative: Hoffmann Eitle

(57) **Abstract**

The present invention provides new therapeutic approaches for the treatment or prevention of FGFR4-expressing cancers, including those resistant to other therapies.

## Description

### Field of the Invention

The present invention relates to the field of cancer. Concretely, the present invention provides new therapeutic approaches for the management of cancers expressing FGFR4.

### Background of the Invention

FGFR4 is a tyrosine kinase receptor involved in the regulation of several cellular phenotypes such as proliferation, differentiation, and survival. Imbalances in FGFR signaling have been shown to have oncogenic roles in many cancers (Turner and Grose, 2010). Interestingly, the function and kinase domain of FGFR4 differs from others FGF receptors, suggesting it may have a unique role when compared to other FGFRs (Beenken and Mohammadi, 2009). Most of the pan-FGFR inhibitors, however, fall short in their ability to competitively bind FGFR4 due to its structural dissimilarity to FGFR1-3.

Hormone receptor-positive (HR+) tumors represent 75% of all breast cancer, are clinically and biologically heterogeneous and are a major cause of cancer death. Several clinical trials have demonstrated improved progression-free survival (PFS) and overall survival (OS) in metastatic HR+ breast cancer patients upon combinations of endocrine therapy with cyclin-dependent kinase 4 and 6 (CDK4/6) inhibition (CDK4/6i), using the inhibitors palbociclib (Cristofanilli et al., 2016; Finn et al., 2016; Turner et al., 2018), ribociclib (Hortobagyi et al., 2018; Im et al., 2019; Slamon et al., 2018; Tripathy et al., 2018), or abemaciclib (Goetz et al., 2017; Johnston et al., 2019; Sledge et al., 2017, 2020). These studies have led to the approval of CDK4/6i as therapeutics in this setting. However, virtually all advanced patients will eventually progress due to the development of CDK4/6i resistance mechanisms. And there is an urgent need to identify such mechanisms of resistance to this new standard of care.

Targeted therapies like antibody-drug conjugates (ADCs) represent a promising approach to overcome resistance to current treatments and provide new therapeutic options. To date, a number of ADCs targeting breast cancer tumors have been approved. However, only one ADC (ENHERTU; trastuzumab deruxtecan) has been approved to treat a subset of HR+ patients, specifically patients with advanced (unresectable or metastatic) HR+/HER2-low BC (Modi et al., NEJM 2022). Although this ADC has significantly improved survival, patients ultimately experience disease progression and this is an unmet patient population. The latest data show that the proportion of patients who progress while on trastuzumab deruxtecan is 50% in less than 10 months and up to 80% in less than 24 months (Modi et al., NEJM 2022).

In view of the above, therefore, there is still the need of further approaches to efficiently manage cancer.

### SUMMARY OF THE INVENTION

The present inventors have found that FGFR4 (fibroblast growth factor receptor 4) is expressed in cancer patients with primary or acquired CDK4/6i resistance.

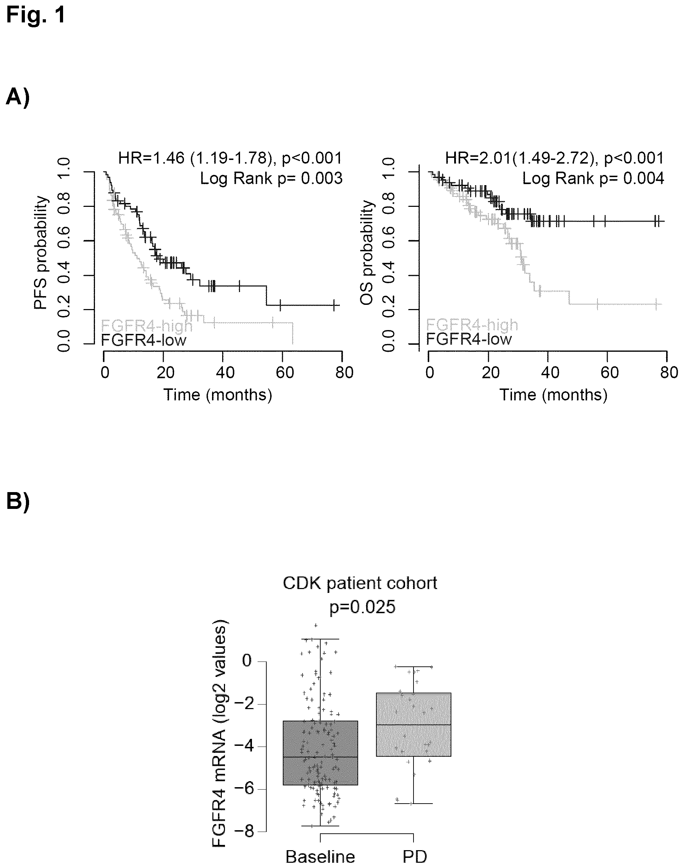

As shown below, the inventors found that in a clinical cohort of metastatic HR+/HER2-negative breast cancer patients treated with CDK4/6i (including palbociclib, ribociclib or abemaciclib) plus endocrine therapy in the 1st, 2nd or successive lines of treatment at Hospital Clinic of Barcelona, (hereafter called the CDK cohort, including baseline samples - both primary and metastatic, as well as progressive samples), higher FGFR4 expression at baseline correlated with worse PFS and OS in CDK4/6i treated patients independently of prior lines of therapy (Fig. 1A). In addition, expression of FGFR4 was significantly higher in progression disease (PD) samples compared to baseline samples (Fig. 1B). These data support the robustness of FGFR4 as an informative marker of response to CDK4/6i therapy, independently of the particular nature of the cancer, the stage or the particular prior therapy.

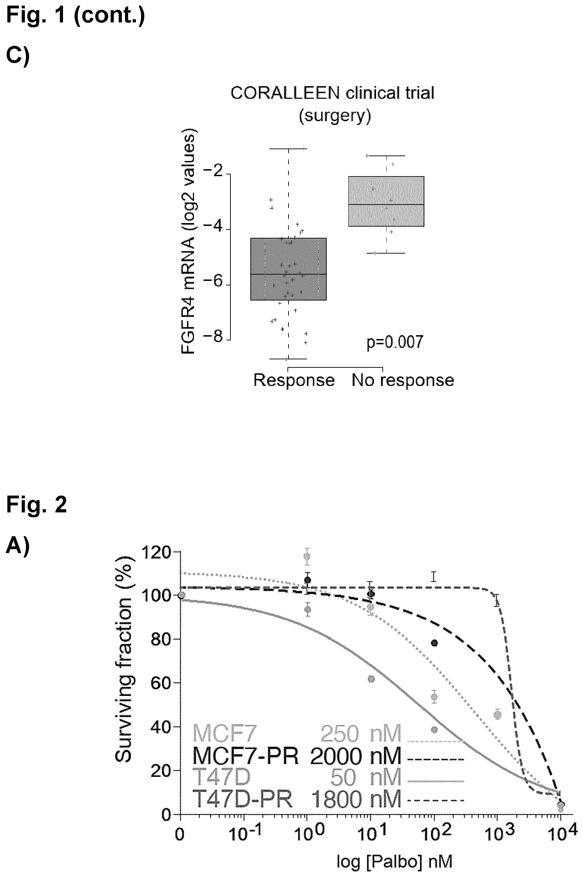

The inventors characterized baseline, day 15 and surgery samples of 49 post-menopausal patients with HR+/HER2-negative and luminal B by PAM50 early-stage breast cancer treated with CDK4/6i ribociclib plus letrozole (estrogen therapy) in the context of the CORALLEEN clinical trial (Prat et al., 2020). Data in Fig. 1C show that higher expression of FGFR4 was observed in surgical samples from non-responder subjects who have already been treated with CDK4/6i compared to responders. These data are therefore indicative that FGFR4 is highly expressed in cases wherein the CDK4/6i therapy is not efficient.

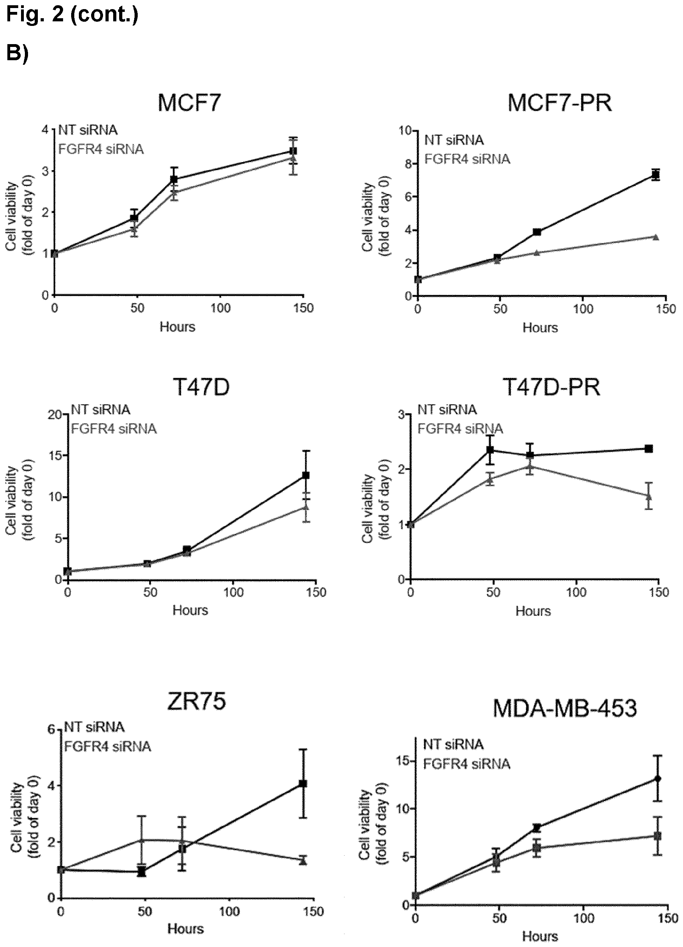

The relation between FGFR4 and the resistance/sensitivity to a CDK4/6i therapy was further elucidated performing FGFR4 siRNA-mediated knockdown in CDK4/6i (palbociclib)-resistant and sensitive cell lines. As it is shown below, see Fig. 2B-D, FGFR4 silencing significantly impaired the growth of all CDK4/6i-resistant cell lines. Not only that, but also, there was a remarkable increase in the IC₅₀ values for all the CDK4/6i-resistant cell lines (MCF7-PR, T47D-PR) compared to the parental cell lines (MCF-7, T47D, Fig. 2A).

From these data it can be concluded that the absence of response to CDK4/6i in cell lines is due to the high expression of FGFR4. Therefore, FGFR4 can provide valuable information to predict, prior to start the particular medical regimen, whether the subject will positively respond to a CDK4/6i-based therapy: if FGFR4 expression level is high, with respect to a reference value, this will indicate that the subject's tumor is resistant to the therapy.

In addition to the above, the information provided by the marker also is indicative of the degree of resistance: the higher the expression with respect to the reference value, the higher the resistance to the treatment.

Altogether this signifies a great advance in the management of CDK4/6i-based therapies because the oncologist, determining the level of FGFR4, either in a tissue biopsy or biological fluid sample, can easily and in early stages, determine whether the subject will positively respond and to what extent.

Thus, in a first aspect the present invention provides a method for determining the response of a subject already diagnosed of having cancer to a medical regimen comprising one or more CDK4/6i, the method comprising the steps of: (a) determining the level of expression of FGFR4 in an isolated test sample of the subject, and (b) comparing with a reference value; wherein if FGFR4 is overexpressed, then this is indicative that there will be a reduced or absence of response to the one or more CDK4/6i.

In a second aspect the present invention provides the use of FGFR4 as a marker for predicting or monitoring the response of a subject to a medical regimen comprising one or more CDK4/6i.

In a third aspect the present invention provides the use of means for determining the level of expression of FGFR4 in a method as defined in the first aspect of the invention.

In a fourth aspect the present invention provides a CDK4/6i for use in a method of treating or preventing cancer in a subject, wherein the method comprises: (i) determining the expression level of FGFR4 in an isolated test sample of the subject; (ii) comparing with a reference value; and (ii) administering a therapeutically effective amount of the CDK4/6i to the subject if the level of expression of FGFR4 in the test sample is equal or lower than the one in the reference value. This aspect can alternatively be formulated as a method of treating or preventing cancer, the method comprising (a) determining the level of expression of FGFR4 in an isolated test sample of the subject, (b) comparing with a reference value; and (c) administering a therapeutically effective amount of a CDK4/6i to the subject if FGFR4 is equal or lower than the reference value.

In a fifth aspect, the invention is directed to a method of treating or preventing cancer, e.g. breast cancer in a subject, e.g. a human, in need thereof by discontinuing administration of a CDK4/6i to said subject after said subject has been determined to have higher FGFR4 expression in the cancer cells compared to a reference value.

The relation between FGFR4 and the resistance/sensitivity to a CDK4/6i therapy was further elucidated performing FGFR4 siRNA-mediated knockdown in CDK4/6i (palbociclib)-resistant and sensitive cell lines. As it is shown below, see Fig. 2B-D, FGFR4 silencing significantly impaired the growth of all CDK4/6i-resistant cell lines. Not only that, but also, there was a remarkable increase in the IC₅₀ values for all the CDK4/6i-resistant cell lines (MCF7-PR, T47D-PR) compared to the parental cell lines (MCF-7, T47D, Fig. 2A). MDA-MB-453 cell line was used as positive control of reported high FGFR4.

Using several *in vitro* and *in vivo* models, provided below, the inventors have demonstrated that FGFR4 not only can provide valuable information regarding the response to CDK4/6i-based therapy, but that FGFR4 can itself be a therapeutic target to overcome resistance to CDK4/6i, and gain efficiency in the treatment of cancer.

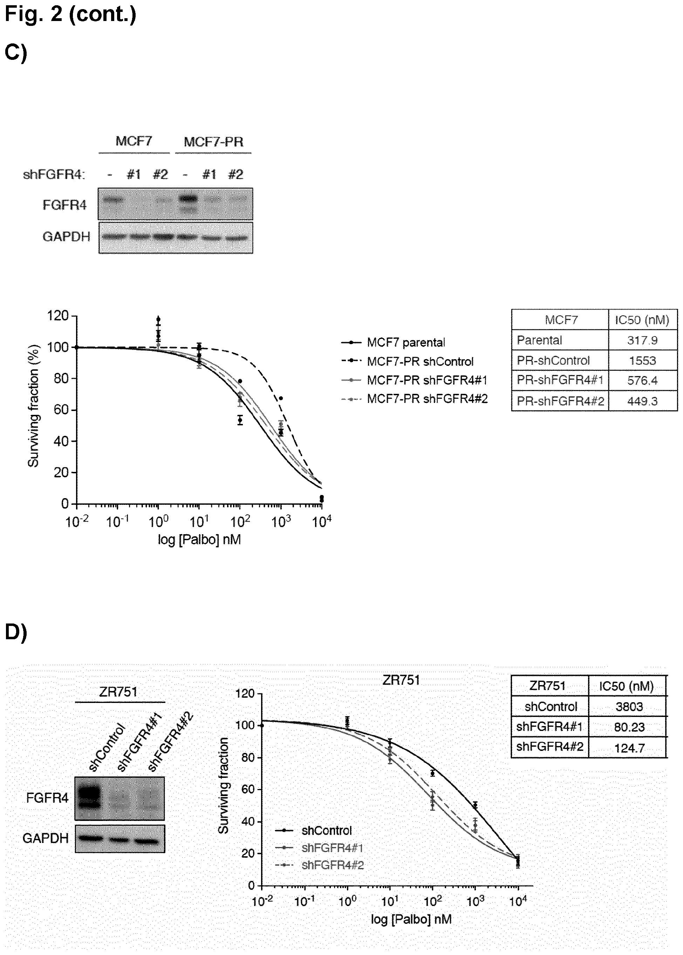

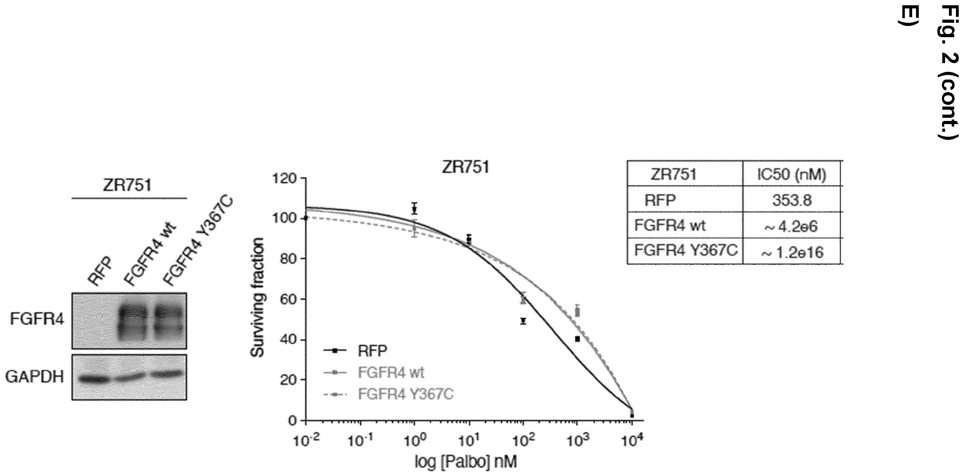

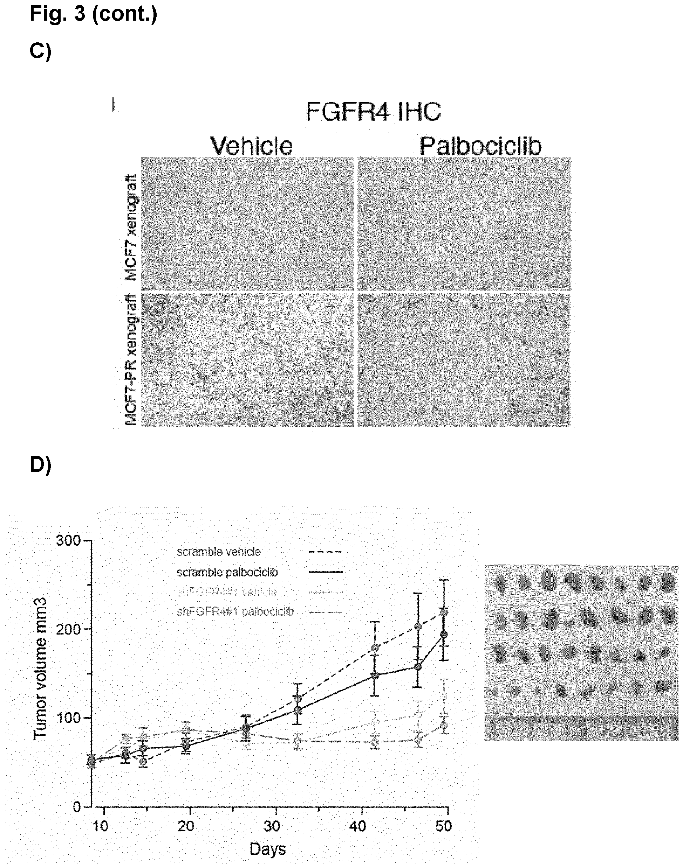

As discussed in more detail *infra,* it was found that cell lines with acquired resistance to CDK4/6i were susceptible to FGFR4 knockdown; in line with this, FGFR4 knockdown re-sensitized breast cancer cell lines to CDK4/6i *in vitro* (Fig. 2). Importantly, these effects were confirmed in an *in vivo* breast cancer model by using MCF7-palbociclib resistant cells (called MCF7-PR) and a FGFR4 knockdown, treated with or without the CDK4/6i palbociclib. FGFR4 gene downregulation reduced tumor growth in a CDK4/6i-resistant breast cancer model and also re-sensitized tumors to CDK4/6i palbociclib *in vivo* in the resistant breast cancer model (Fig. 3D). Therefore, FGFR4 inactivation restores CDK4/6i response and prevents tumor growth.

Thus, in a sixth aspect the present invention provides a FGFR4 inhibitor for use in reducing the resistance to CDK4/6i(s) in a subject suffering from cancer, by reducing FGFR4 expression. This aspect can alternatively be formulated as the use of a FGFR4 inhibitor for the manufacture of a medicament for reducing the resistance to CDK4/6i (s) in a subject suffering from cancer. This aspect can alternatively be formulated as a method for reducing the resistance to CDK4/6i (s), in a subject suffering from cancer, the method comprising administering a therapeutically effective amount of a FGFR4 inhibitor to the subject in need thereof. In an embodiment, the FGFR4 inhibitor is an anti-FGFR4 antibody or anti-FGFR4 antibody-drug conjugate.

The invention also provides in a seventh aspect a FGFR4 inhibitor for use in the treatment of cancer in a subject resistant to CDK4/6i. This aspect can be formulated as the use of a FGFR4 inhibitor for the manufacture of a medicament for the treatment of cancer in a subject resistant to CDK4/6. This aspect can also be formulated as a method for the treatment of cancer in a subject resistant to CDK4/6i, the method comprising administering a therapeutically effective amount of a FGFR4 inhibitor to the subject in need thereof.

Due to its relevance in cancer, the present inventors also addressed the design of an efficient, therapeutic approach for subjects suffering from cancer correlating with expression of FGFR4. FGFR4 could be used then as a marker for cancer cells, hence as a hook to specifically bring and deliver the cytotoxic therapy to the tumoral cells, e.g., breast tumoral cells, correlating with the expression of FGFR4.

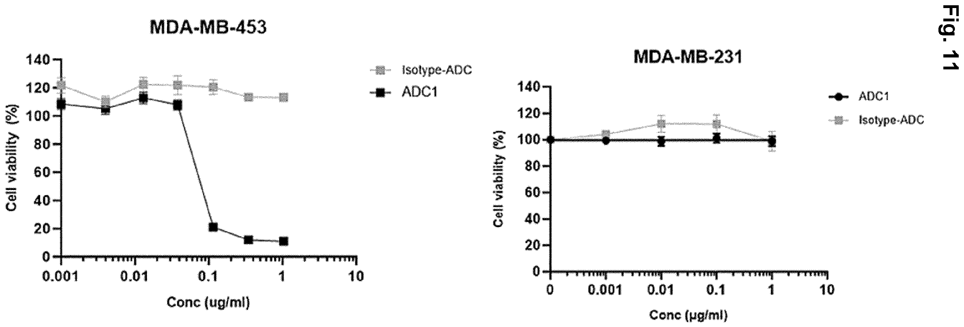

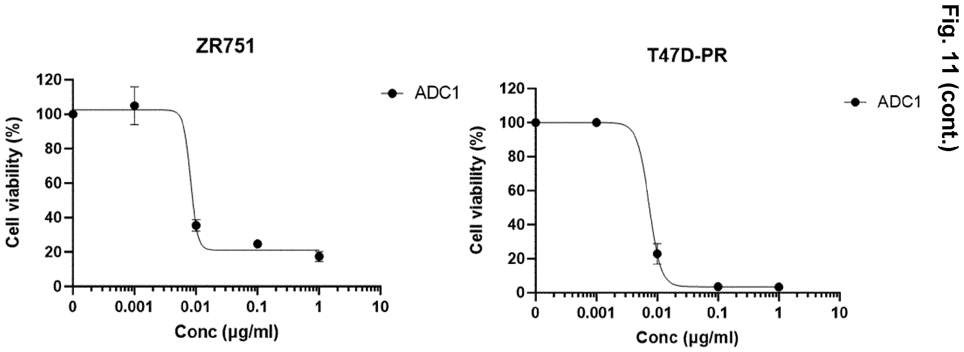

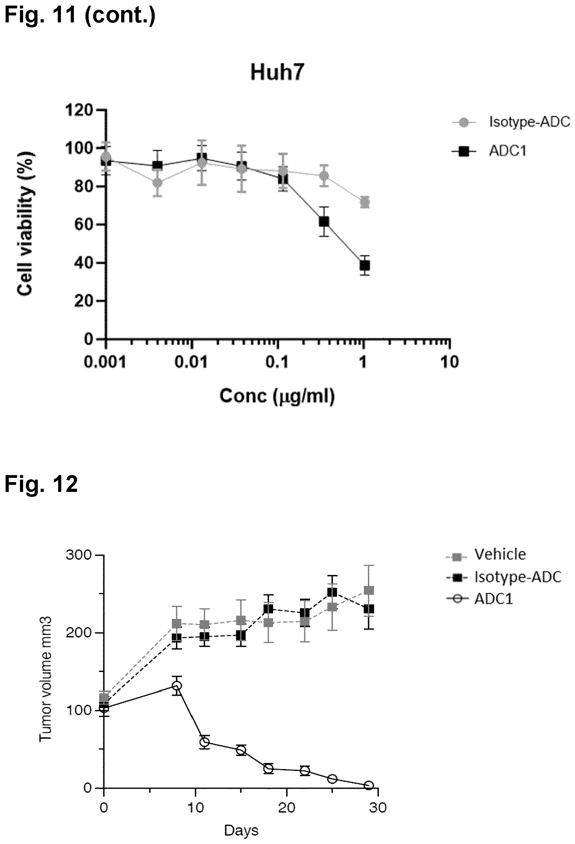

Thus, the inventors have found that anantibody-drug conjugate (ADC) including FGFR4-selective antibodies were highly efficient in killing several FGFR4-expressing tumoral cell lines (Fig.11).

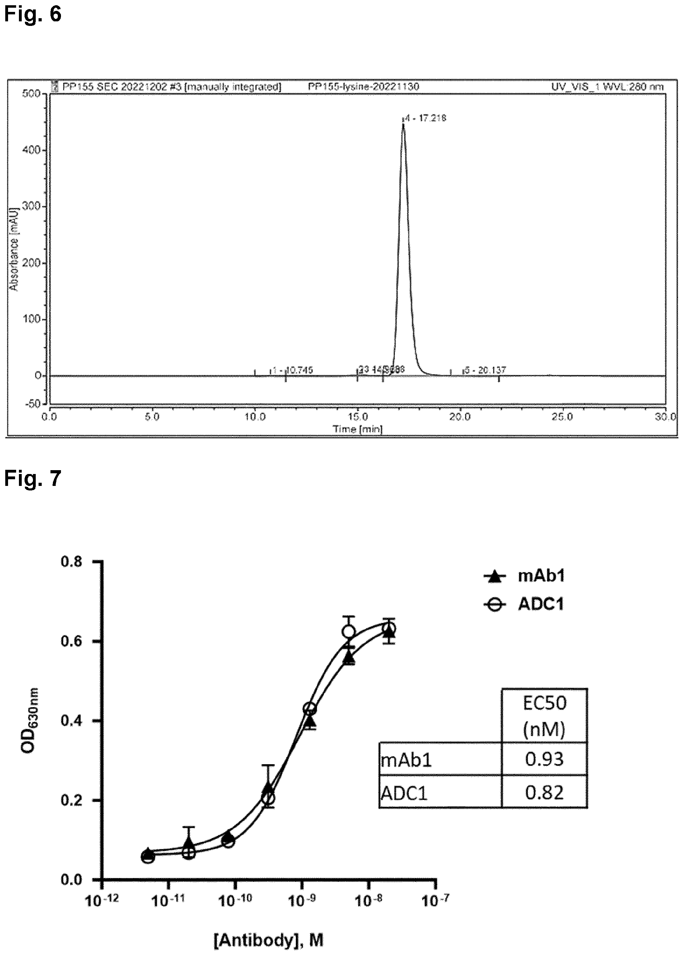

In addition, the inventors found that an anti-FGFR4ADC comprising an anti-FGFR4 antibody which did not bind FGFR1, FGFR2 and FGFR3 retained the high binding affinity to the target (if compared to the naked antibody), and showed to be highly stable (when the PK profile was determined), safe (no significant non-specific binding towards DNA, insulin and heparin was detected), and efficient as anti-cancer agent in cell lines and in vivo. See the Examples Fig 6-12).

That is, conjugating the anti-FGFR4 antibody with specificity towards FGFR4 to a drug by a linker, the resulting ADC is highly stable, safe, and effective. Something which means a great advance in the field of cancer therapy, and especially in the development of efficient and safe ADCs for the treatment of cancer.

Here, the toxic payload of the ADC is the driver of the efficacy of the therapy.

In view of the above, in an eighth aspect the present invention provides an anti-FGFR4 antibody drug conjugate, wherein the anti-FGFR4 antibody:
- directly binds to FGFR4, and
- is not specific of FGFR1, FGFR2, and FGFR3.

In a ninth aspect the present invention provides a pharmaceutical composition comprising the ADC as defined in the eighth aspect of the invention, together with one or more pharmaceutically acceptable excipients and/or carriers.

In a tenth aspect the present invention provides the anti-FGFR4 ADC or the pharmaceutical composition of the invention as defined herein, for use in the treatment or prevention of a cancer expressing FGFR4. This aspect can also be formulated as the use of anti-FGFR4 ADC or the pharmaceutical composition as defined herein for the manufacture of a medicament for the treatment or prevention of a cancer expressing FGFR4. This aspect can also be formulated as a method for the treatment or prevention of a cancer expressing FGFR4, the method comprising administering a therapeutically effective amount of the anti-FGFR4 ADC or the pharmaceutical composition of the invention.

In an eleventh aspect the present invention provides a method for deciding or recommending initiating the administration of an ADC as defined herein, for a subject which suffers from cancer, the method comprising the step of determining the level of expression of FGFR4; wherein, if it is confirmed the expression of FGFR4, then it is recommended the administration of the ADC as defined herein.

In a final aspect, the invention is directed to a method of treating cancer in a subject, e.g., a human, in need thereof, the method comprising administering a therapeutically effective amount of an anti-FGFR4 antibody-drug conjugate to said subject wherein said subject has previously been determined to have higher expression of FGFR4 in the cancer cells compared to a reference expression value. In one embodiment, the subject has previously failed treatment with a CDK4/6 inhibitor. In another embodiment, the subject discontinued treatment with CDK4/6i after being determined to have high expression of FGFR4 in the cancer cells and prior to administration of an anti-FGFR4 ADC.

### BRIEF DESCRIPTION OF THE DRAWINGS

Fig. 1. (A) FGFR4 expression was associated with worse PFS and OS response to CDK4/6i treatment in CDK clinical cohort. (B) FGFR4 gene expression in baseline and PD samples in CDK cohort; (C) FGFR4 gene expression after letrozole (estrogen therapy) plus ribociclib (CDK4/6i) treatment comparing those tumors that responded vs those that did not respond.
Fig. 2. A) Dose-response to the CDK4/6i palbociclib in T47D and MCF7 parental and resistant to palbociclib (T47D-PR and MCF7-PR). (B) Growth dynamics of each cell line after FGFR4 silencing. (C) upper, protein validation by WB of FGFR4 depletion upon expression of an FGFR4 short hairpin construct (1 or 2) or a control scramble shRNA in MCF7 and MCF7-PR cells; bottom, dose response to palbociclib on indicated control and FGFR4-depleted cell lines. (D) Left, protein validation by WB of FGFR4 depletion upon expression of an FGFR4 short hairpin construct (1 or 2) or a control scramble shRNA in ZR751 cells; right, dose response to palbociclib on indicated control and FGFR4-depleted cell lines. (E) Left, protein validation by WB of FGFR4 expression; right, dose-response to palbociclib in parental ZR751, overexpressing FGFR4 (wt) and overexpressing a constitutively active form of FGFR4 (Y367C).
Fig. 3 (A) Growth rate of MCF7 control cells (left) and MCF7-PR cells (right), upon vehicle placebo or CDK4/6i treatment. (B) FGFR4 mRNA level and proliferation score of xenograft tumors from (A). (C) FGFR4 expression by immunohistochemistry in samples from (A). (D) Left, Tumor growth of control and FGFR4-depleted MCF7-PR cells in the presence or absence of CDK4/6i treatment. Right, representative pictures of tumors at the end of the experiment.
**Fig. 4****.** Binding selectivity of mAb1 and mAb2 for FGFR4 versus the other FGFRs,
**Fig. 5****.** Cytotoxicity mediated by FGFR4 antibodies in breast and hepatocellular cancer cell lines.
**Fig. 6****.** Size-exclusion chromatography of the ADC1 exemplified in below sections.
**Fig. 7****.** Binding ELISA of the naked mAb1 antibody and of ADC1.
**Fig. 8****.** Serum stability of the naked mAb1 antibody and of ADC1,
**Fig. 9****.** Binding to DNA, heparin and insulin of the naked mAb1 antibody and of ADC1.
**Fig. 10****.** Pharmacokinetic evaluation of the naked mAb1 antibody and of ADC1 .
**Fig. 11****.** Cytotoxic activity of ADC1 in several breast and hepatocellular cancer cell lines expressing different levels of FGFR4.
**Fig. 12****.** In vivo efficacy of ADC1 in a breast cancer model expressing FGFR4.

### DETAILED DESCRIPTION OF THE INVENTION

Terms not specifically defined herein should be given the meanings that would be given to them by one of skill in the art in light of the disclosure and the context. As used in the specification, however, unless specified to the contrary, the following terms have the meaning indicated and the following conventions are adhered to.

Throughout the present specification and the accompanying claims, the words "comprise" and variations such as "comprises", "comprising" are to be interpreted inclusively. That is, these words are intended to convey the possible inclusion of other elements or integers not specifically recited, where the context allows. The word "comprise" also includes the term "consists of".

The present invention provides in a first aspect a method for determining the response of a subject already diagnosed of having cancer to a medical regimen comprising one or more CDK4/6i, the method comprising:
(a) determining the level of expression of FGFR4 in an isolated test sample of the subject, and
(b) comparing with a reference value,
wherein if the FGFR4 expression level is higher than the reference value, then this is indicative of a reduced or absence of response to the medical regimen comprising the one or more CDK4/6i.

The term "FGFR4" is a member of the fibroblast growth factor receptor superfamily. FGFR family members differ from one another in their ligand affinities and tissue distribution. A full-length representative protein would consist of an extracellular region, composed of three immunoglobulin-like domains, a single hydrophobic membrane-spanning segment and a cytoplasmic tyrosine kinase domain. The extracellular portion of the protein interacts with fibroblast growth factors, setting in motion a cascade of downstream signals, ultimately influencing mitogenesis and differentiation. The genomic organization of FGFR4, compared to members 1-3, encompasses 18 exons rather than 19 or 20. Although alternative splicing has been observed, there is no evidence that the C-terminal half of the Iglll domain of this protein varies between three alternate forms, as indicated for members 1-3. Splice forms are also encompassed by "FGFR4", in the context of the invention. In one embodiment, the FGFR4 is human FGFR4. Human FGFR4 sequences (including variants and isoforms) are already known in the state of the art and are encompassed by the present invention. Illustrative non-limitative examples of human FGFR4 are provided in GeneBank (Gene ID: 2264, updated on 9-Jul-2023) and UniProt (P22455, 2001-04-27 v2).

In the context of the invention the "determining of response" encompasses both the prediction of the response prior to start, for the first time or in subsequent therapeutic cycles, the medical regimen; as well as the monitoring of the evolution of the subject during the treatment, i.e., whether the subject is suffering the onset of the resistance and, if positive, how severe the resistance is.

The term "cancer" as used herein refers to a malignant neoplasm characterized by deregulated or unregulated cell growth. In one embodiment of the invention, the cancer is selected from oral squamous cell carcinoma, head and neck cancer, esophageal cancer, gastric cancer, ovarian cancer, cervical cancer, lung cancer, breast cancer, renal cancer, prostate cancer, sarcoma, melanoma, leukemia, lymphoma, kidney cancer, duodenum cancer, small intestine cancer, large intestine cancer, colorectal cancer, liver cancer, pancreatic cancer, bladder cancer, or cholangiocarcinoma; particularly the cancer is breast cancer, liver cancer, gastric cancer or colorectal cancer. In a particular embodiment the cancer is breast cancer.

In the context of the present invention, "CDK4/6 inhibitors" (also referred as "CDK4/6i") refers to molecules that provide an inhibitory effect CDK4/6. CDK4/6 are serine-threonine kinases that regulate cell cycle progression. They interact with cyclin D, leading to the phosphorylation of the retinoblastoma (Rb) protein, which in turn leads to progression through the G1 checkpoint to the S phase of the cell cycle. ER signaling can upregulate cyclin D1 levels and activate CDK4/6 signaling pathways. Inhibition of CDK4/6 prevents cell cycle progression and induces cancer cells to exit the cell cycle into a senescent or quiescent state. Thus, in the context of the invention "CDK4/6 inhibitor" refers to any agent that inhibits the kinase activity of CDK4 and CDK6.

In certain embodiments, the CDK4/6i may be derived from pyridopyrimidine, pyrrolopyrimidine or indolocarbazole compounds.

In certain embodiments, the CDK4/6i may be palbociclib (PD0332991), LEE-011 (ribociclib), LY2835219 (abemaciclib), G1T28-1, SHR6390, or P276-00, or a derivative of any one of palbociclib, LEE-011, LY2835219, G1T28-1, SHR6390, or P276-00. In certain embodiments the CDK4/6i is palbociclib.

The terms "palbociclib" or "PD-0332991" (commercial or trade name Ibrance) generally refer to a selective CDK4 and CDK6 inhibitor.

The terms "LEE-011" or "ribociclib" generally refer to a selective CDK4 and CDK6 inhibitor having CAS Number 1211441-98-3.

The terms "LY2835219" or "Abemaciclib" generally refer to an oral selective CDK4 and CDK6 inhibitor having Cas Number 1231929-97-7.

The term "G1T28-1" refers to a selective CDK4 and CDK6 inhibitor developed and tested by G1 Terapeutics Inc.

The term "SHR6390" refers to a selective CDK4 and CDK6 inhibitor developed and tested by Jiangsu HengRui Medicine Co.

The term "P276-00" refers to a selective CDK4 and CDK6 inhibitor having CAS Number 920113-03-7.

In certain embodiments, the CDK4/6i may comprise or consist of antisense oligonucleotides, shRNA molecules and/or siRNA molecules that specifically inhibit the expression or activity of CDK4 and/or CDK6. One non-limiting example of a CDK4/6i comprises an antisense, shRNA, or siRNA nucleic acid sequence which is substantially identical (i.e., largely but not wholly identical) to the sequence of at least a portion of a CDK4 or CDK6 nucleic acid sequence, wherein the identity of the portion relative to the CDK4 or CDK6 sequence is at least about 75%, or at least about 80%, or at least about 85%, or at least about 90%, or at least about 95%, or at least about 98%, where percent identity can be determined as known in the art, for example, BLAST or FASTA software. Sequence identity may be determined using suitable algorithms for performing sequence alignments and determination of sequence identity as know per se. Exemplary but non-limiting algorithms include those based on the Basic Local Alignment Search Tool (BLAST) originally described by Altschul et al. 1990 (J Mol Biol 215: 403-10), such as the "Blast 2 sequences" algorithm, for example using the published default settings or other suitable settings (such as, e.g., for the BLASTN algorithm: cost to open a gap = 5, cost to extend a gap = 2, penalty for a mismatch = -2, reward for a match = 1, gap × dropoff = 50, expectation value = 10.0, word size = 28; or for the BLASTP algorithm: matrix = Blosum62, cost to open a gap = 11, cost to extend a gap = 1, expectation value = 10.0, word size = 3).

In certain embodiments, the complementary portion may constitute at least 10 nucleotides, or at least 15 nucleotides or at least 20 nucleotides, or at least 25 nucleotides, or at least 30 nucleotides, and the antisense nucleic acid, shRNA or siRNA molecules may be up to 15 nucleotides, or up to 20 nucleotides, or up to 25 nucleotides, or up to 30 nucleotides, or up to 35 nucleotides, or up to 40 nucleotides, or up to 45 nucleotides, or up to 50 nucleotides, or up to 75 nucleotides, or up to 100 nucleotides in length.

In the context of the invention, the term "expression" or "level of expression", is understood to be the presence, detectable by standard techniques known in the state of the art, of a protein or a messenger RNA.

In the context of the invention, the term "higher expression", alternatively referred to as "overexpression", is understood to be the presence, detectable by standard techniques known in the state of the art, of a protein or a messenger RNA, above a reference value compared with a reference sample or in relation to the median of a reference population.

In the context of the invention, the term "reference sample" is understood as the sample from a healthy subject. In one embodiment, the cancer is breast cancer and the reference sample is the breast tissue sample of a healthy subject.

In the present invention, the term "reference value" is to be understood as a predefined value of a given molecular marker, which is derived from the levels of said molecular marker in a sample or group of samples. If the level of expression is determined at the protein level, then the "reference value" is a predefined value of protein quantity, whereas if the level of expression is determined at the mRNA level, then the "reference value" is a predefined value of mRNA quantity. The samples are taken from a subject or group of subjects wherein the presence, absence, stage, or course of the disease has been properly performed previously. This value is used as a threshold in order to discriminate subjects wherein the condition to be analyzed is present from those wherein such condition is absent.

The subject or subjects from whom the "reference value" is derived may include subject/s wherein the condition is absent, subject/s wherein the condition is present, or both. The skilled person in the art, making use of the general knowledge, is able to choose the subject or group of subjects more adequate for obtaining the reference value for each of the methods of the present invention. Methods for obtaining the reference value from the group of subjects selected are well-known in the state of the art. In one embodiment, the reference value is determined in a sample or group of samples from healthy subject(s).

In one embodiment of the methods of the invention, the level of expression of FGFR4 is determined prior to start the medical regimen. Thus, FGFR4 can be used as a predictive biomarker to determine whether the subject will respond to the therapy, either because the subject is going to be administered for the first time with the CDK4/6 inhibitors or the subject is going to repeat the CDK4/6 medical regimen already provided in other phases of the disease. In a particular embodiment of the first aspect of the invention, the subject to which the analysis is performed, is going to be administered, for the first time, with the medical regimen comprising the one or more CDK4/6i(s). In an alternative embodiment, the subject has already been treated with one or more CDK4/6i(s) prior to determining the level of expression of FGFR4. In a further alternative embodiment of the first aspect of the invention, the level of expression of FGFR4 is determined during the CDK4/6i medical regimen, in order to monitor the onset of the CDK4/6i resistance (when an increase in FGFR4 expression is detected).

In another embodiment of the first aspect of the invention, the medical regimen further comprises an endocrine therapy.

In the context of the invention, the term "endocrine therapy" is to be understood as any therapy designed to interrupt the signal generated by estrogen binding to estrogen receptor. Non-limiting examples include treatments which lower the estrogen level and treatments designed to block the estrogen receptor, such as administration of the drug tamoxifen.

In another embodiment of the first aspect of the invention, determining the level of expression of FGFR4 comprises determining the amount of FGFR4 mRNA.

The level of expression of FGFR4 mRNA may be determined by quantitative real-time PCR (qPCR), reverse transcription-qPCR (RT-qPCR), a nucleic acid microarray, digital molecular barcoding technology (nCounter Nanostring), branched DNA (bDNA) signal amplification technology (Quantigene), mass spectrometry (Sequenom), or a combination of said methods. Suitable reagents and kits are already available and the skilled person just has to follow manufacturer's instructions. Illustrative examples are provided below.

Alternatively, the level of expression of FGFR4 is determined at protein level. In this embodiment, the protein marker includes, but is not limited to, native-sequence polypeptides, isoforms, chimeric polypeptides, all homologs, fragments, and precursors of the markers, including modified forms of the polypeptides and derivatives thereof.

In any of the embodiments provided above or below, the level of expression can be determined by immunochemistry.

The term "immunochemistry" as used herein refers to a variety of techniques for detecting antigens (usually proteins and peptides, and in the present case any of the proteins listed above alone or in combination) in a sample by exploiting the principle of antibodies binding specifically to said antigens.

Visualizing an antibody-antigen interaction can be accomplished in a number of ways. In the most common instance, an antibody is conjugated to an enzyme, such as peroxidase, that can catalyse a colour-producing reaction.

Alternatively, the antibody can also be tagged to a fluorophore, such as fluorescein or rhodamine. The immunochemistry technique can be direct or indirect. The direct method is a one-step staining method and involves a labeled antibody (e.g. FITC-conjugated antiserum) reacting directly with the antigen. While this technique utilizes only one antibody and therefore is simple and rapid, the sensitivity is lower due to little signal amplification, such as with indirect methods, and is less commonly used than indirect methods. The indirect method involves an unlabeled primary antibody (first layer) that binds to the target antigen in the sample and a labeled secondary antibody (second layer) that reacts with the primary antibody. This method is more sensitive than direct detection strategies because of signal amplification due to the binding of several secondary antibodies to each primary antibody if the secondary antibody is conjugated to the fluorescent or enzyme reporter.

Further amplification can be achieved if the secondary antibody is conjugated to several biotin molecules, which can recruit complexes of avidin-, streptavidin or Neutravidin-enzyme. The indirect method, aside from its greater sensitivity, also has the advantage that only a relatively small number of standard conjugated (labeled) secondary antibodies needs to be generated.

With the direct method, it would be necessary to label each primary antibody for every antigen of interest. It must be borne in mind that immunochemistry techniques can also be used to detect certain nucleic acid sequences if a tagged nucleic acid probe (designed to specifically bind to a certain target nucleic acid sequence) can later be detected with a labelled antibody.

Thus, the detection of the protein could be performed by using a tagged nucleic acid designed to bind a specific sequence of the target protein RNA, and then detecting said tagged nucleic acid with a labelled antibody which selectively binds to the tag.

Immunoassay procedures suitable include enzyme-linked immunosorbent assays (ELISA), enzyme immunodot assay, agglutination assay, antibody-antigen-antibody sandwich assay, antigen-antibody-antigen sandwich assay, immunochromatography, or other immunoassay formats well-known to the ordinarily skilled artisan.

In another embodiment of the methods of the invention, the level of expression of protein is determined using an antibody or a fragment thereof able to bind to the target.

In another particular embodiment of the methods of the invention, the means to carry out the invention form part of a kit. The antibody or fragment thereof for detecting the target protein(s) can be included in a kit. The kit may additionally comprise means (additives, solvents) to visualize the antibody-protein interactions.

In another embodiment of the methods of the invention, the test sample is selected from a biological fluid sample (such as serum, blood, plasma), a tissue sample (such as a tissue homogenate) or a cell lysate.

In one embodiment, the sample is a biopsy. The term "biopsy" generally denotes a sample of cells or tissues removed from a living subject for examination. The biopsy may be an excisional biopsy (i.e., when an entire lump or suspicious area is removed), an incisional biopsy or core biopsy (i.e., when only a sample of tissue is removed with preservation of the histological architecture of the tissue's cells), or a needle aspiration biopsy (i.e., when a sample of tissue or fluid is removed with a needle in such a way that cells are removed without preserving the histological architecture of the tissue cells).

In any of the embodiments provided above or below, the level of expression is determined in liquid biopsy, including but not limited to circulating tumor cells, circulating tumor DNA or thereof.

The sample can be subjected to a variety of well-known post-collection preparative and storage techniques (e. g. fixation, storage, freezing, lysis, homogenization, DNA or RNA extraction, ultrafiltration, concentration, evaporation, centrifugation, etc.) prior to determining the level of expression in the sample.

In a particular embodiment of the invention, the sample may be a formalin-fixed paraffin-embedded (FFPE) sample or fresh frozen sample. Preferably, the sample is a FFPE sample. The present methods advantageously allow determining the sensitivity of a human subject having breast cancer to a CDK4/6i in FFPE samples which are routinely used in the clinic.

In one embodiment, the methods of the invention further comprise (i) collecting the result of the comparison (as responder or non-responder), and (ii) saving the information in a data carrier. In the sense of the invention a "data carrier" is to be understood as any means that contain meaningful information data for the diagnosis or prognosis of endometrial carcinoma, such as paper. The carrier may also be any entity or device capable of carrying the prognosis data. For example, the carrier may comprise a storage medium, such as a ROM, for example a CD ROM or a 5 semiconductor ROM, or a magnetic recording medium, for example a floppy disc or hard disk. Further, the carrier may be a transmissible carrier such as an electrical or optical signal, which may be conveyed via electrical or optical cable or by radio or other means. When the prognosis data are embodied in a signal that may be conveyed directly by a cable or other device or means, the 10 carrier may be constituted by such cable or other device or means. Other carriers relate to USB devices and computer archives. Examples of suitable data carrier are paper, CDs, USB, computer archives in PCs, or sound registration with the same information.

In a third aspect the present invention provides the use of means for determining the level of expression of FGFR4 in a method as defined in the first aspect of the invention. All the embodiments provided under the first aspect of the invention, related to the determination of the FGFR4 expression, are also embodiments of the third aspect of the invention.

In a fourth aspect the present invention provides a CDK4/6i for use in a method of treating or preventing cancer in a subject, wherein the method comprises: (i) determining the expression level of FGFR4 in an isolated test sample of the subject; (ii) comparing with a reference control value; and (ii) administering a therapeutically effective amount of a CDK4/6i to the subject if the level of expression of FGFR4 in the test sample is equal to or lower than the one in the reference value. All the embodiments provided above, under the first aspect of the invention, are also embodiments of the fourth aspect of the invention.

In the context of the invention, "equal to the reference control value" encompasses not statistically significant differences between the expression level of the test sample and the reference value.

The terms "treat" or "treatment" encompass both the therapeutic treatment of an already developed disease or condition, such as the therapy of an already developed cancer, as well as prophylactic or preventive measures, wherein the aim is to prevent or lessen the chances of incidence of an undesired affliction, such as to prevent occurrence, development and progression of cancer. Beneficial or desired clinical results may include, without limitation, a reduction in tumor burden or a decrease in the number of size of metastases, a diminishment of extent of disease, stabilised (i.e., not worsening) state of disease, delay or slowing of disease progression, amelioration or palliation of the disease state, and the like. "Treatment" can also mean prolonging survival as compared to expected survival if not receiving treatment. "Amelioration" refers to the reduction in the number or severity of signs or symptoms of cancer.

As used in the present invention, the term "prevention" or "preventing" refers to inhibiting the full development of a disease.

The methods provided by the invention allow to administer a therapeutically effective amount of the CDK4/6i as taught herein in subjects having cancer, particularly breast cancer, including HR(+)- breast cancer, which will benefit from such treatment.

The term "therapeutically effective amount" as used in the context of the invention refers to an amount of active compound that elicits the biological or medicinal response in a subject that is being sought by a surgeon, researcher, veterinarian, medical doctor or other clinician, which may include inter alia alleviation of the symptoms of the disease or condition being treated. Methods are known in the art for determining therapeutically effective doses of the compound (either the CDK4/6i or the FGFR4 inhibitor, depending on the particular aspect). In one embodiment, a therapeutically effective amount is the amount necessary to eliminate, reduce the size, or prevent metastasis of a tumor.

Appropriate therapeutically effective doses of the CDK4/6i as taught herein may be determined by a qualified physician with due regard to the nature of the CDK4/6i, the disease condition and severity, and the age, size and condition of the patient.

Without limitation, a typical dose of the CDK4/6i, for instance palbociclib, to be administered may range from about 10 mg to about 1000 mg per administration. For example, the dose of the CDK4/6i, for instance palbociclib, to be administered may range from about 100 mg to about 500 mg or from about 75 mg to about 200 mg, per administration. Alternatively, a CDK4/6i palbociclib may be administered by intravenous administration (typically 50 mg intravenous dose) as an infusion (e.g., 4-hours infusion).

Further for instance, a CDK4/6i such as ribociclib (LEE011) may be orally administered daily 3 weeks on/1 week off in 28-day cycles (50-600 mg, typically 600 mg). For instance, a CDK4/6i such as LY2835219 (typically 150-200 mg) may be administered orally once every 12 hours in 21- or 28-day cycles.

In a sixth aspect the present invention provides a FGFR4 inhibitor for use in reducing the resistance to CDK4/6i(s).

As used herein, a "FGFR4 inhibitor" is an entity which inhibits FGFR4. Preferably, the FGFR4 inhibitor is a selective FGFR4 inhibitor and, for example, it is a compound that inhibits FGFR4 selectively compared to FGFR1, FGFR2 and FGFR3. FGFR4 inhibitors may be small molecules or large molecules, such as antibodies or conjugates including antibodies.

In one embodiment of the sixth aspect of the invention, the FGFR4 inhibitor is a small molecule. There are well-known small molecules in the state of the art with the ability of inhibiting FGFR4, particularly of irreversibly inhibiting FGFR4. Illustrative non-limitative examples of FGFR4 inhibitors are: Fisogatinib (Blu-554; Blueprint Medicines), H3B-6527 (N-[2-[[6-[[[(2,6-dichloro-3,5-dimethoxyphenyl)amino]carbonyl]methylamino]-4-pyrimidinyl]amino]-5-(4-ethyl-1-piperazinyl)phenyl]-2-propenamide; CAS number 1702259-66-2; H3 Biomedicine), Roblitinib (FGF40, CAS No. 1708971-55-4; Novartis), INCB062079 (Ruggeri B. et al., 2017), and U3-1784 (WO 2016/023894, Daiichi Sankyo).

In an alternative embodiment of the sixth aspect of the invention, the FGFR4 inhibitor is a blocking antibody. Illustrative non-limitative examples of these antibodies are provided in in the following patent applications WO2009/009173, WO2007/136893, WO2012/138975, WO2010/026291, WO2008/052798, WO2010/004204, WO2014/105849, WO2014/165287 and WO2016/023894, WO2014/144737, WO2014/145909, WO2014/011900, WO2015/057963, WO2015/057938, WO2015/030021, WO2015/107171, WO2015/059668, WO2016/064960, WO2016/134320, WO2016/134314, WO2016/134294, the entire contents of each of which is incorporated herein by reference.

In the context of the invention, the term "reducing the resistance to CDK4/6i" means that the cancer cells, once treated with the FGFR4 inhibitor, partially or totally recover the sensitivity (i.e., respond) to CDK4/6i. Protocols for determining the reduction in the resistance are well-known in the state of the art.

In one embodiment of the sixth aspect of the invention, the FGFR4 inhibitor is administered to a subject who is already receiving a CDK4/6i-based therapy, either simultaneously, consecutively or separately. In an alternative embodiment of the sixth aspect of the invention, the FGFR4 inhibitor is administered to a patient who is not receiving any CDK4/6i therapy, yet.

In an eighth aspect the present invention provides an ADC comprising an anti-FGFR4 antibody which selectively binds to FGFR4.

In the context of the invention, the term "antibody" is an immunoglobulin molecule capable of specific binding to a target, such as a carbohydrate, polynucleotide, lipid, polypeptide, etc., through at least one antigen recognition site, located in the variable region of the immunoglobulin molecule. As used herein, unless otherwise indicated by context, the term is intended to encompass not only intact polyclonal or monoclonal antibodies, but also fragments thereof (such as Fab, Fab', F(ab')₂, Fv), and domain antibodies, including shark and camelid antibodies), and fusion proteins comprising an antibody portion, multivalent antibodies (e.g., COVX-BODY^{™}), multispecific antibodies (e.g., bispecific antibodies so long as they exhibit the desired biological activity) and antibody fragments as described herein, and any other modified configuration of the immunoglobulin molecule that comprises an antigen recognition site. An antibody includes an antibody of any class, such as IgG, IgA, or IgM (or sub-class thereof), and the antibody need not be of any particular class. Depending on the antibody amino acid sequence of the constant domain of its heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1 and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known. On one embodiment, the immunoglobulin is a human, murine, monkey, or rabbit immunoglobulin. In another embodiment the "antibody" is a full-length immunoglobulin or a fragment thereof (such as Fab, Fab', F(ab')₂, Fv).

The term "Fab containing polypeptide" as used herein refers to a polypeptide comprising a Fab fragment, Fab' fragment, or "(Fab')₂ fragment." A Fab-containing polypeptide may comprise part or all of a wild-type hinge sequence (generally at the carboxyl terminus of the Fab portion of the polypeptide). A Fab-containing polypeptide may be obtained or derived from any suitable immunoglobulin, such as from at least one of the various IgG1, IgG2, IgG3, or IgG4 subtypes, or from IgA, IgE, IgD or IgM. A Fab-containing polypeptide may be a Fab-containing fusion polypeptide, wherein one or more polypeptides are linked to a Fab-containing polypeptide. A Fab fusion combines the Fab polypeptide of an immunoglobulin with a fusion partner, which in general may be any protein, polypeptide, or small molecule. Virtually any protein or small molecule may be linked to the Fab polypeptide to generate a Fab-containing fusion polypeptide. Fab-containing fusion partners may include, but are not limited to, the target-binding region of a receptor, an adhesion molecule, a ligand, an enzyme, a cytokine, a chemokine, or some other protein or protein domain.

A "Fab fragment" is comprised of one light chain and the CH1 and variable regions of one heavy chain. The heavy chain of a Fab molecule cannot form a disulphide bond with another heavy chain molecule.

A "Fab' fragment" contains one light chain and a portion of one heavy chain that contains the VH domain and the CH1 domain and the region between the CH1 and CH2 domains, such that an interchain disulphide bond can be formed between the two heavy chains of two Fab' fragments to form a F(ab')₂ molecule.

A "F(ab')₂ fragment" contains two light chains and two heavy chains containing a portion of the constant region between the CH1 and CH2 domains, such that an interchain disulfide bond is formed between the two heavy chains. A F(ab')₂ fragment thus is composed of two Fab' fragments that are held together by a disulfide bond between the two heavy chains.

"Antibody fragments" as used herein comprise only a portion of an intact antibody, wherein the portion preferably retains at least one, preferably most or all, of the functions normally associated with that portion when present in an intact antibody.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigen. Further, in contrast to polyclonal antibody preparations that typically include different antibodies directed against different determinants (epitopes), each monoclonal antibody is directed against a single determinant on the antigen.

There are well known means in the state of the art for preparing and characterizing antibodies. Methods for generating polyclonal antibodies are well known in the prior art. Briefly, one prepares polyclonal antibodies by immunizing an animal with the protein; then, serum from the immunized animal is collected and the antibodies isolated. A wide range of animal species can be used to produce the antiserum. Typically, the animal used for production of antisera can be a rabbit, mouse, rat, hamster, guinea pig or goat.

Moreover, monoclonal antibodies (MAbs) can be prepared using well-known techniques. Typically, the procedure involves immunizing a suitable animal with the protein associated with the disease. The immunizing composition can be administered in an amount effective to stimulate antibody producing cells.

Methods for preparing monoclonal antibodies are initiated generally following the same lines as the polyclonal antibody preparation. The immunogen is injected into animals as antigen. The antigen may be mixed with adjuvants such as complete or incomplete Freund's adjuvant. At intervals of two weeks, approximately, the immunization is repeated with the same antigen.

The monoclonal antibodies herein may, in certain embodiments, specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US4816567).

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit, or nonhuman primate having the desired specificity, affinity, and capacity. In some instances, framework region (FR) residues of the human immunoglobulin are replaced by corresponding non-human residues. Humanized antibodies may, moreover, comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin.

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

In the context of the eighth aspect of the invention the anti-FGFR4, the antibody is one which binds to FGFR4 and does not bind to FGFR1, FGFR2 and FGFR3, as referred above. That is, the anti-FGFR4 antibody does not have any statistically significant effect on the binding to FGFR1, FGFR2 and FGFR3. A variety of immunoassay formats may be used to select antibodies specifically binding to FGFR4. For example, solid-phase ELISA immunoassays are routinely used to select antibodies specifically immunoreactive with a protein (see, e.g., Hnasko, ELISA: Methods and Protocols (2015) Humana Press, chapter 6, p.61-69), for a description of immunoassay formats and conditions that can be used to determine specific immunoreactivity).

In the context of the invention the expression "binds to FGFR4" when referred to anti-FGFR4 antibody, means that the antibody or fragment thereof interacts (and binds) with its epitope, whether that epitope is linear or conformational. "does not bind to FGFR1, FGFR2, and FGFR3", means that the anti-FGFR4 does not significantly bind to any of these targets. The expression "significantly bind"

In the context of the invention, the term "linker" is to be understood as a bifunctional or multifunctional moiety that can be used to link one or more drug moieties to an antibody to form an ADC.

In one embodiment, the ADC provided by the present invention includes a cleavable linker. Cleavable linkers are preferably stable extracellularly in a sufficient manner to be therapeutically effective. Before transport or delivery into a cell, the ADC is preferably stable and remains intact, i.e. the antibody remains conjugated to the drug moiety. Linkers that are stable outside the target cell may be cleaved at some efficacious rate once inside the cell. Thus, an effective linker will: (i) maintain the specific binding properties of the antibody; (ii) allow delivery, e.g., intracellular delivery, of the drug moiety; and (iii) maintain the therapeutic effect, e.g., cytotoxic effect, of a drug moiety.

Illustrative non-limitative examples of "cleavable linker," include acid-labile linkers (e.g., comprising hydrazone), protease-sensitive (e.g., peptidase-sensitive) linkers, photolabile linkers, or disulphide-containing linkers (US5208020) (herein incorporated by reference in its entirety). A cleavable linker is typically susceptible to cleavage under intracellular conditions. Suitable cleavable linkers include, for example, a peptide linker cleavable by an intracellular protease, such as lysosomal protease or an endosomal protease. In exemplary embodiments, the linker can be a dipeptide linker, such as a valine-citrulline (val-cit) or a phenylalanine-lysine (Phe-Lys) linker.

In one embodiment, the linker is cleavable under intracellular conditions, such that cleavage of the linker sufficiently releases the drug from the antibody in the intracellular environment to be therapeutically effective. In some embodiments, the cleavable linker is pH-sensitive, i.e., sensitive to hydrolysis at certain pH values. Typically, the pH-sensitive linker is hydrolyzable under acidic conditions. For example, an acid-labile linker that is hydrolyzable in the lysosome (e.g., a hydrazone, semicarbazone, thiosemicarbazone, cis-aconitic amide, orthoester, acetal, ketal, or the like) can be used. (See, e.g., U.S. Pat. Nos. 5122368; 5824805; 5622929; for instance, herein incorporated by reference in its entirety). Such linkers are relatively stable under neutral pH conditions, such as those in the blood, but are unstable at below pH 5.5 or 5.0, the approximate pH of the lysosome. In certain embodiments, the hydrolyzable linker is a thioether linker (such as, e.g., a thioether attached to the therapeutic agent via an acylhydrazone bond (see, e.g., US5622929, herein incorporated by reference in its entirety).

In other embodiments, the linker is cleavable under reducing conditions (e.g., a disulfide linker). A variety of disulfide linkers are known in the art, including, for example, those that can be formed using SATA (N-succinimidyl-5-acetylthioacetate), SPDP (N-succinimidyl-3-(2-pyridyldithio)propionate), SPDB (N-succinimidyl-3-(2-pyridyldithio)butyrate) and SMPT (N-succinimidyloxycarbonyl-alpha-methyl-alpha-(2-pyridyl-dithio)toluene), SPDB and SMPT (US4880935, herein incorporated by reference in its entirety).

In some embodiments, the linker is cleavable by a cleaving agent, e.g., an enzyme, that is present in the intracellular environment (e.g., within a lysosome or endosome or caveolea). The linker can be, e.g., a peptidyl linker that is cleaved by an intracellular peptidase or protease enzyme, including, but not limited to, a lysosomal or endosomal protease. In some embodiments, the peptidyl linker is at least two amino acids long or at least three amino acids long. Cleaving agents can include cathepsins B and D and plasmin, all of which are known to hydrolyze dipeptide drug derivatives resulting in the release of active drug inside target cells. Most typical are peptidyl linkers that are cleavable by enzymes that are present in EGFR-expressing cells. Examples of such linkers are described, e.g., in US6214345, incorporated herein by reference in its entirety and for all purposes. In a specific embodiment, the peptidyl linker cleavable by an intracellular protease is a Val-Cit linker or a Phe-Lys linker (see, e.g., US6214345, herein incorporated by reference in its entirety, which describes the synthesis of doxorubicin with the Val-cit linker). One advantage of using intracellular proteolytic release of the therapeutic agent is that the agent is typically attenuated when conjugated and the serum stabilities of the conjugates are typically high.

In an alternative embodiment, the linker is a non-cleavable linker. Non-cleavable linkers prevent release of the cytotoxic agent before the ADC is internalized by the target cell. Once in the lysosome, digestion of the antibody by lysosomal proteases results in the release of the cytotoxic agent.

In the context of the invention, the ADC includes one or more drugs. In some embodiments, the drug is a small molecule. In some examples, the drug is a cross-linking agent, an anti-microtubule agent and/or anti-mitotic agent, or any cytotoxic agent suitable for mediating killing of tumor cells. Exemplary cytotoxic agents include, but are not limited to, a PDB, an auristatin, a maytansinoid, dolastatin, calicheamicin, nemorubicin and its derivatives, PNU-159682, anthracycline, vinca alkaloid, taxane, trichothecene, CC1065, camptothecin, elinafide, a combretastain, a dolastatin, a duocarmycin, an enediyne, a geldanamycin, an indolino-benzodiazepine dimer, a puromycin, a tubulysin, a hemiasterlin, a spliceostatin, or a pladienolide, as well as stereoisomers, isosteres, analogs, and derivatives thereof that have cytotoxic activity.

Anti-FGFR4 antibodies may be conjugated to at least one auristatin. Auristatins represent a group of dolastatin analogs that have generally been shown to possess anticancer activity by interfering with microtubule dynamics and GTP hydrolysis, thereby inhibiting cellular division. For example, Auristatin E (US5635483, herein incorporated by reference in its entirety) is a synthetic analogue of the marine natural product dolastatin 10, a compound that inhibits tubulin polymerization by binding to the same site on tubulin as the anticancer drug vincristine. Dolastatin 10, auristatin PE, and auristatin E are linear peptides having four amino acids, three of which are unique to the dolastatin class of compounds. Exemplary embodiments of the auristatin subclass of mitotic inhibitors include, but are not limited to, monomethyl auristatin D (MMAD or auristatin D derivative), monomethyl auristatin E (MMAE or auristatin E derivative), monomethyl auristatin F (MMAF or auristatin F derivative), auristatin F phenylenediamine (AFP), auristatin EB (AEB), auristatin EFP (AEFP), and 5-benzoylvaleric acid-AE ester (AEVB). The synthesis and structure of auristatin derivatives are described, for instance, in WO04010957 or WO02088172, among others, herein incorporated by reference in its entirety.

In one embodiment, anti-FGFR4 antibodies are conjugated to at least one MMAE (monomethyl auristatin E). Monomethyl auristatin E (MMAE, vedotin) inhibits cell division by blocking the polymerization of tubulin. Because of its super toxicity, it also cannot be used as a drug itself. In recent cancer therapy developments, it is linked to a monoclonal antibody (mAb) that recognizes a specific marker expression in cancer cells and directs MMAE to the cancer cells.

In another embodiment, the drug is a pyrrolobenzodiazepine (PBD). As one example, PDB dimers recognize and bind to specific DNA sequences, and have been shown to be useful as cytotoxic agents. PBD dimers have been conjugated to antibodies and the resulting ADC shown to have anti-cancer properties (see, for example, US 2010/0203007, herein incorporated by reference in its entirety). Exemplary linkage sites on the PBD dimer include the five-membered pyrrolo ring, the tether between the PBD units, and the N10-C11 imine group (see WO 2009/016516; US 2009/304710; US 2010/047257; US 2009/036431; US 2011/0256157; and WO 2011/130598, herein incorporated by reference in its entirety).

In another embodiment, the drug is a maytansinoid molecule. Maytansinoids are derivatives of maytansine, and are mitototic inhibitors which act by inhibiting tubulin polymerization.

In another embodiment, the ADC comprises one or more calicheamicin molecules. The calicheamicin family of antibiotics, and analogues thereof, are capable of producing doublestranded DNA breaks at sub-picomolar concentrations (Hinman et al., Cancer Res 53:3336-3342, 1993). Exemplary methods for preparing ADCs with a calicheamicin drug moiety are described in U.S. Pat. Nos. 5,712,374; 5,714,586; 5,739,116; and 5,767,285, herein incorporated by reference in its entirety .

In some embodiments, the ADC comprises an anthracycline. Anthracyclines are antibiotic compounds that exhibit cytotoxic activity. It is believed that anthracyclines can operate to kill cells by a number of different mechanisms, including intercalation of the drug molecules into the DNA of the cell thereby inhibiting DNA-dependent nucleic acid synthesis; inducing production of free radicals which then react with cellular macromolecules to cause damage to the cells; and/or interactions of the drug molecules with the cell membrane. Non-limiting exemplary anthracyclines include doxorubicin, epirubicin, idarubicin, daunomycin, daunorubicin, doxorubicin, epirubicin, nemorubicin, valrubicin and mitoxantrone, and derivatives thereof. For example, PNU-159682 is a potent metabolite (or derivative) of nemorubicin (Quintieri et al., Clin Cancer Res 11(4):1608-1617, 2005). Nemorubicin is a semisynthetic analog of doxorubicin with a 2-methoxymorpholino group on the glycoside amino of doxorubicin (Grandi et al., Cancer Treat Rev 17:133, 1990; Ripamonti et al., Br J Cancer 65:703-707, 1992).

In another embodiments the drug is a radioactive isotope to generate cytotoxic radiopharmaceuticals, referred to as radioimmunoconjugates. Examples of radioactive isotopes that can be conjugated to antibodies for use diagnostically or therapeutically include, but are not limited to, iodine-131, indium-111, yttrium-90, and lutetium-177. Methods for preparing radioimmunoconjugates are established in the art. Examples of radioimmunoconjugates are commercially available, including Zevalin^{®} (DEC Pharmaceuticals) and Bexxar^{®} (Corixa Pharmaceuticals), and similar methods can be used to prepare radioimmunoconjugates using the antibodies of the invention. In certain embodiments, the macrocyclic chelator is 1,4,7,10-tetraazacyclododecane-N,N',N",N" '-tetraacetic acid (DOTA) which can be attached to the antibody via a linker molecule. Such linker molecules are commonly known in the art and described in Denardo et al., (1998) Clin Cancer Res. 4(10):2483-90; Peterson et al., (1999) Bioconjug. Chem. 10(4):553-7; and Zimmerman et al., (1999) Nucl. Med. Biol. 26(8):943-50, each incorporated by reference in their entireties.

The ADCs suitable in the context of the invention can be prepared following any of the protocols well-known to those skilled in the art. For example, a cysteine thiol of an antibody can form a bond with a reactive functional group of a linker or a drug-linker intermediate to make an ADC. In one embodiment, the conjugates of the present invention can be prepared by conjugating pre-formed drug-linker compound to the antibody or fragment thereof, as described, for instance, in US6441163, US2011/0003969, and US2008/0145374, followed by a purification step.

In a ninth aspect the present invention provides a pharmaceutical composition.

The precise therapeutic dose of the ADC of the invention, may depend on several variables. Some of these would be: route of administration, time of drug release (e.g., instant or extended), administration schedule, pain severity, condition of the patient, and the like. The pharmaceutical compositions can be prepared as a liquid, semi-solid or solid dosage form, for example in the form of solutions for injection, drops, juices, syrups, sprays, suspensions, tablets, patches, capsules, dressings, suppositories, ointments, creams, lotions, gels, emulsions, aerosols or in multiparticulate form, for example in the form of pills or granules, if appropriate compressed into tablets, decanted into capsules or suspended in a liquid, or administered as such.

These compositions can be prepared with the aid of conventional means, devices, methods or processes known in the art.

Pharmaceutically acceptable adjuvants, vehicles or excipients which may be used in such compositions are adjuvants, vehicles or excipients known to those skilled in the art or commonly used in the preparation of therapeutic compositions, which may be selected, for example, from the group consisting of excipients, fillers, solvents, diluents, surfactants, colorants, preservatives, disintegrants, sliding agents, lubricants, flavoring agents or binders.

The term "pharmaceutically acceptable" refers to pharmaceutically acceptable materials, compositions, or vehicles. Each component must be pharmaceutically acceptable in the sense of being compatible with the other ingredients of the pharmaceutical composition. It must also be suitable for use in contact with the tissue or organ of humans without excessive toxicity, irritation, allergic response, immunogenicity or other problems or complications commensurate with a reasonable benefit/risk ratio in animals and, particularly, in humans.

The selection of physiologically compatible adjuvants or the number of adjuvants to be used depends on the form of administration of the pharmaceutical composition, i.e., oral, subcutaneous, parenteral, intravenous, intraperitoneal, intradermal, intramuscular, intranasal, buccal, rectal, otic or intratympanic. Preparations in the form of tablets, dragees, capsules, granules, pills, drops, in particular otic drops, juices or syrups are preferably suitable for oral administration; solutions, suspensions, easy to reconstitute dry preparations or also sprays are preferably suitable for parenteral, topical or inhalation administration. The compounds in accordance with the invention used in the pharmaceutical composition in accordance with the invention in a depot, in a dissolved form or in a dressing, or if appropriate having added other agents favouring penetration into the skin, are preparations suitable for percutaneous administration. The preparation forms administrable orally or percutaneously can also release the respective compound according to the invention in a delayed form.

For instance, for oral administration in the form of a tablet or capsule, the active drug components can be combined with an oral, non-toxic, pharmaceutically acceptable, inert carrier such as lactose, starch, sucrose, glucose, methyl cellulose, magnesium stearate, dicalcium phosphate, calcium sulphate, mannitol, sorbitol and the like; for oral administration in liquid form, the oral drug components can be combined with any oral, non-toxic, pharmaceutically acceptable inert carrier such as ethanol, glycerol, water, and the like. Moreover, when desired or necessary, suitable binders, lubricants, disintegrating agents, and colouring agents can also be incorporated into the mixture. Suitable binders include starch, gelatine, natural sugars such as glucose or beta-lactose, corn sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate, and the like. Disintegrators include, without limitation, starch, methyl cellulose, agar, bentonite, xanthan gum, and the like.

Gelatine capsules contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain colouring and flavouring to increase patient acceptance.

The dosage administered of the pharmaceutical composition will, of course, vary depending on the use and known factors such as the age, health, and weight of the recipient; nature and extent of symptoms, concurrent treatments, if any, frequency of treatment, and the effect desired. The recipient may be any type of mammal, but is preferably a human.

In one embodiment of the invention, the pharmaceutical composition is lyophilized.

In a tenth aspect the invention provides the use of the ADC herein provided, in any of the embodiments provided above, in the treatment of a cancer expressing FGFR4. Illustrative non-limitative examples of cancers expressing FGFR4 are oral squamous cell carcinoma, head and neck cancer, esophageal cancer, gastric cancer, ovarian cancer, cervical cancer, lung cancer, breast cancer, renal cancer, prostate cancer, sarcoma, melanoma, leukemia, lymphoma, kidney cancer, duodenum cancer, small intestine cancer, large intestine cancer, colorectal cancer, liver cancer, pancreatic cancer, bladder cancer, or cholangiocarcinoma; particularly the cancer is breast cancer, liver cancer, gastric cancer or colorectal cancer. In a particular embodiment the cancer expressing FGFR4 is breast cancer.

In the context of the invention, the cancer is one wherein there is FGFR4 expression, independently of the level of expression of FGFR1, FGFR2, or FGFR3. In the context of the invention, the therapeutic effect is due to the selective effect on FGFR4.

In one embodiment, the cancer correlates with an overexpression of FGFR4 (i.e., FGFR4 is expressed above the level found in non-cancer population). Protocols and means to determine the level of expression of FGFR4 in an isolated sample of the subject are well-known (as explained above and illustrated below).

In one embodiment of the seventh aspect of the invention, the subject suffering cancer is furthermore resistant to a medical regimen comprising one or more CDK4/6i.

In another embodiment of the invention, the ADC is administered in combination with other therapeutic agents, such as other anti-cancer agents, such as chemotherapeutic agents, such as, for example, mitotic inhibitors, alkylating agents, antimetabolites, intercalating antibiotics, growth factor inhibitors, cell cycle inhibitors, enzymes, topoisomerase inhibitors, anti-survival agents, biological response modifiers, endocrine therapy, immunomodulators and anti-angiogenesis agents; anti-allergic agents; anti-nausea agents (or anti-emetics); pain relievers; cytoprotective agents; and combinations thereof. Other anti-cancer treatments include radiation therapy.

General Chemotherapeutic agents considered for use in combination therapies include anastrozole (Arimidex^{®}), bicalutamide (Casodex^{®}), bleomycin sulfate (Blenoxane^{®}), busulfan (Myleran^{®}), busulfan injection (Busulfex^{®}), capecitabine (Xeloda^{®}), N4-pentoxycarbonyl-5-deoxy-5-fluorocytidine, carboplatin (Paraplatin^{®}), carmustine (BiCNU^{®}), chlorambucil (Leukeran^{®}), cisplatin (Platinol^{®}), cladribine (Leustatin^{®}), cyclophosphamide (Cytoxan^{®} or Neosar^{®}), cytarabine, cytosine arabinoside (Cytosar-U^{®}), cytarabine liposome injection (DepoCyt^{®}), dacarbazine (DTIC-Dome^{®}), dactinomycin (Actinomycin D, Cosmegan), daunorubicin hydrochloride (Cerubidine^{®}), daunorubicin citrate liposome injection (DaunoXome^{®}), dexamethasone, docetaxel (Taxotere^{®}), doxorubicin hydrochloride (Adriamycin^{®}, Rubex^{®}), etoposide (Vepesid^{®}), fludarabine phosphate (Fludara^{®}), 5- fluorouracil (Adrucil^{®}, Efudex^{®}), flutamide (Eulexin^{®}), tezacitibine, Gemcitabine (difluorodeoxycitidine), hydroxyurea (Hydrea^{®}), Idarubicin (Idamycin^{®}), ifosfamide (IFEX^{®}), irinotecan (Camptosar^{®}), L-asparaginase (ELSPAR^{®}), leucovorin calcium, melphalan (Alkeran^{®}), 6-mercaptopurine (Purinethol^{®}), methotrexate (Folex^{®}), mitoxantrone (Novantrone^{®}), mylotarg, paclitaxel (Taxol^{®}), phoenix (Yttrium90/MX-DTPA), pentostatin, polifeprosan 20 with carmustine implant (Gliadel^{®}), , teniposide (Vumon^{®}), 6-thioguanine, thiotepa, tirapazamine (Tirazone^{®}), topotecan hydrochloride for injection (Hycamptin^{®}), vinblastine (Velban^{®}), vincristine (Oncovin^{®}), and vinorelbine (Navelbine^{®}).

Non-limiting examples of alkylating agents include nitrogen mustards (such as mechlorethamine, cyclophosphamide, melphalan, uracil mustard or chlorambucil), alkyl sulfonates (such as busulfan), nitrosoureas (such as carmustine, lomustine, semustine, streptozocin, or dacarbazine).

Non-limiting examples of antimetabolites include folic acid analogs (such as methotrexate), pyrimidine analogs (such as 5-FU or cytarabine), and purine analogs, such as mercaptopurine or thioguanine.

Non-limiting examples of natural products include vinca alkaloids (such as vinblastine, vincristine, or vindesine), epipodophyllotoxins (such as etoposide or teniposide), antibiotics (such as dactinomycin, daunorubicin, doxorubicin, bleomycin, plicamycin, or mitomycin C), and enzymes (such as L-asparaginase).

Non-limiting examples of miscellaneous agents include platinum coordination complexes (such as cis-diamine-dichloroplatinum II also known as cisplatin), substituted ureas (such as hydroxyurea), methyl hydrazine derivatives (such as procarbazine), and adrenocrotical suppressants (such as mitotane and aminoglutethimide).

Non-limiting examples of hormones and antagonists include adrenocorticosteroids (such as prednisone), progestins (such as hydroxyprogesterone caproate, medroxyprogesterone acetate, and magestrol acetate), estrogens (such as diethylstilbestrol and ethinyl estradiol), antiestrogens (such as tamoxifen), and androgens (such as testerone proprionate and fluoxymesterone). Examples of the most commonly used chemotherapy drugs include Adriamycin, Alkeran, Ara-C, BiCNU, Busulfan, CCNU, Carboplatinum, Cisplatinum, Cytoxan, Daunorubicin, DTIC, 5-FU, Fludarabine, Hydrea, Idarubicin, Ifosfamide, Methotrexate, Mithramycin, Mitomycin, Mitoxantrone, Nitrogen Mustard, Taxol (or other taxanes, such as docetaxel), Velban, Vincristine, VP-16, while some more newer drugs include Gemcitabine (Gemzar), Herceptin, Irinotecan (Camptosar, CPT-11), Leustatin, Navelbine, Rituxan STI-571, Taxotere, Topotecan (Hycamtin), Xeloda (Capecitabine), Zevelin and calcitriol.

In one embodiment, the immunomodulators are PD-1/PD-L1 immunomodulators. In some embodiments, the PD-1/PD-L1 immunomodulator is a PD-1 blocker. In some embodiments, the PD-1/PD-L1 immunomodulator is an anti-PD-1 blocking antibody. In some embodiments, the PD-1/PD-L1 immunomodulator is an anti-PD-L1 blocking antibody. PD-1 and PD-L1 therapies are well known in the art and include but are not limited Cemiplimab (Libtayo), Nivolumab (Opdivo), Pembrolizumab (Keytruda), Atezolizumab (Tecentriq), Avelumab (Bavencio), Durvalumab (Imfinzi), Pembrolizumab (Keytruda), Nivolumab (Opdivo), Cemiplimab (Libtayo), Atezolizumab (Tecentriq), Retifanlimab (zynyz), and Dostarlimab (Jemperli), among others.

Other non-limiting examples of immunomodulators that can be used in the context of the invention are AS-101 (Wyeth-Ayerst Labs.), bropirimine (Upjohn), gamma interferon (Genentech), GM-CSF (granulocyte macrophage colony stimulating factor; Genetics Institute), IL-2 (Cetus or Hoffman-LaRoche), human immune globulin (Cutter Biological), IMREG (from Imreg of New Orleans, La.), SK&F 106528, and TNF (tumor necrosis factor; Genentech), among others.

Another common treatment for some types of cancer is surgical treatment, for example surgical resection of the cancer or a portion of it. Another example of a treatment is radiotherapy, for example administration of radioactive material or energy (such as external beam therapy) to the tumor site to help eradicate the tumor or shrink it prior to surgical resection.

Endocrine therapy includes estrogen receptor antagonists such as fulvestrant, raloxifene, tamoxifen, toremifene, arzoxifene, LU 335563 (desmethylarzoxifene), LU 335124, LU 326315, CHE-4227, nafoxidine, lasofoxifene, 21if Llben, 20Ln106, LN10L20 (A-007), TAδ-108, bazedoxyphenacetate (1- {4- [2- (azepan-1-yl) ethoxy] benzyl} -2- (4-hydroxyphenyl) - 3-methyl-1H-indole-5- ol-acetate), EKA-923, afimoxifene, (2) -4-hydroxy tamoxifen, enclomiphene, fispemifen, acolbifen, EM-652, EM-800, droloxifene, idoxifene, OA 5638, TAT-59, OA-7603, centchroman, levoromeloxifene, 1C1-164384, BB-3040, CH-4893237, 8K 16158, 8K 16 137, Kai-1901, 8EKM 3471 (P8K-3471), NMK3339, NMK3656, SS 8490, 11 b-fluoro-7- [5- (methyl-3 - [(4,4,5,5,5- pentafluoropentyl) sulfanyl] propylamino) pentyl] estra-1,3,5 (10) -trien-3,17b-diol (8H 646), 11b-fluoro-17a-methyl-7a-5 [methyl (8,8,9,9,9-pentafluorononyl) amino] pentylestra-1,3,5 (10) -trien-3,17b-diol or (+) - 3 - ( 4-hydroxyphenyl) -2- [4- (2-piperidin-1-ethoxy) phenyl] -4- (trifluoromethyl) -2H-chromen-7-ol, among others.

In one embodiment, the antibody drug conjugate of the present invention is combined in a pharmaceutical combination formulation, or dosing regimen as combination therapy, with a second compound having anti-cancer properties. The second compound of the pharmaceutical combination formulation or dosing regimen can have complementary activities to the immunoconjugate of the combination such that they do not adversely affect each other. For example, the antibody drug conjugate of the present invention can be administered in combination with, but not limited to, a chemotherapeutic agent, a tyrosine kinase inhibitor, a FGF downstream signalling pathway inhibitor, IAP inhibitors, Bcl2 inhibitors, or Mcl1 inhibitors, among others.

In one embodiment of the invention, the ADC is administered in combination with CDK4/6 inhibitor(s). In another embodiment of the invention, the ADC is administered in combination with endocrine therapy. In another embodiment of the invention, the ADC is administered in combination with CDK4/6i(s) and endocrine therapy.

The term "pharmaceutical combination" as used herein refers to either a fixed combination in one dosage unit form, or non- fixed combination or a kit of parts for the combined administration where two or more therapeutic agents may be administered independently at the same time or separately within time intervals.

The term "combination therapy" refers to the administration of two or more therapeutic agents to treat a therapeutic condition or disorder described in the present disclosure. Such administration encompasses co-administration of these therapeutic agents in a substantially simultaneous manner, such as in a single capsule having a fixed ratio of active ingredients.

Alternatively, such administration encompasses co-administration in multiple, or in separate containers (e.g., capsules, powders, and liquids) for each active ingredient. Powders and/or liquids may be reconstituted or diluted to a desired dose prior to administration. In addition, such administration also encompasses use of each type of therapeutic agent in a sequential manner, either at approximately the same time or at different times. In either case, the treatment regimen will provide beneficial effects of the drug combination in treating the conditions or disorders described herein.

The therapeutically effective amount of the ADC or pharmaceutical composition, alone or in combination with any other anti-cancer drug, is understood as defined above, i.e., as the amount that provides the beneficial therapeutic effect.

While the invention has been described in conjunction with specific embodiments thereof, it is evident that many alternatives, modifications, and variations will be apparent to those skilled in the art considering the foregoing description.

The herein disclosed aspects and embodiments of the invention are further supported by the following non-limiting examples.

### EXAMPLES

### I. Relation of FGFR4 with CDK4/6i resistance

### FGFR4 is a potential driver of resistance to CDK4/6 inhibition

FGFR4 gene expression was evaluated in a clinical cohort of patients over the age of 18 years with a histologic diagnosis of HR+/HER2-negative metastatic breast cancer treated with CDK4/6 inhibitors, including palbociclib, ribociclib and abemaciclib at Hospital Clinic of Barcelona between years 2014 and 2021 (CDK cohort). In this cohort, 134 baseline samples (including 61 primary and 73 metastatic) and 28 progressive disease (PD) samples were available for molecular characterization. For that, RNA was extracted using the High Pure FFPE RNA isolation kit (Roche) following manufacturer's protocol and macrodissection was performed, when needed, to avoid normal tissue contamination. A minimum of 100 ng of total RNA was analyzed at the nCounter platform (Nanostring Technologies) using the Breast Cancer 360 Panel, which measures the expression of 771 breast cancer-related genes and 5 housekeeping genes (*ACTB, MRPL19, PSMC4, RPLP0,* and *SF3A1*)*.* Gene expression of *FGFR4* in baseline samples was the top gene significantly associated with worse progression-free survival (PFS; hazard ratio [HaR] [95% confidence intervals (Cl)]=1.46 [1.19-1.78], p<0.001) and overall survival (OS; HaR [95% Cl] = 2.01 [1.49-2.72], p<0.001) in univariate analysis **(****Fig. 1A****).** PFS was defined as the period between start of the treatment with endocrine therapy and CDK4/6i treatment and the time of progression. OS was defined as the period between start of the treatment with endocrine therapy and CDK4/6i treatment and death or last follow-up. Estimates of survival were from the Kaplan-Meier curves and tests of differences by the log-rank test. Univariate and multivariable Cox models were used to test the prognostic significance of each variable. All differences were considered significant at p-value (p)<0.05. In addition, expression of *FGFR4* was significantly higher in samples from patients with progressive disease (PD) compared to baseline samples **(****Fig. 1B****).**

The inventors also evaluated FGFR4 expression following the methodology described above in surgical samples from the ribociclib and letrozole arm in the context of the CORALLEEN phase II trial (NCT03248427; Prat et al. 2020). The inventors characterized baseline, day 15 and surgery samples of 49 post-menopausal women with HR+/HER2-negative early-stage breast cancer. This was a trial in postmenopausal women with primary operable HR+/HER2-negative which randomized patients (1:1) to receive either six 28-days cycles of ribociclib (oral 600 mg once daily for 3 weeks on, 1 week off) plus daily letrozole (oral 2.5 mg/day) or four cycles of doxorubicin (intravenous 60 mg/m²) and cyclophosphamide (intravenous 600 mg/m²) every 21 days followed by weekly paclitaxel (intravenous 80 mg/m²) for 12 weeks. The total duration of the neoadjuvant therapy was 24 weeks and the primary endpoint was the rate of PAM50 Risk of Relapse (ROR)-low disease at surgery in each arm. Higher expression of *FGFR4* was observed in the surgical samples of non-responders compared to responders to CDK4/6i (**Fig. 1C**). These results suggest a positive selection of FGFR4 through the treatment process and a correlation with the non-responder phenotype.

### Functional validation of FGFR4 as a driver of resistance to CDK4/6 inhibition

The inventors next generated experimental cell lines models with acquired resistance to palbociclib derived from commercially available breast cancer cell lines MCF7 and T47D (ATCC), which were cultured according to supplier's recommendation. An MCF7-derived palbociclib-resistant (MCF7-PR) and a T47D-derived palbociclib-resistant (T47D-PR) cell lines were established by treating MCF7 and T47D with palbociclib for 6 months, starting at low concentration (1 nM) and increasing them at each passage until reaching 1 µM). MCF7-PR and T47D-PR cells were maintained in complete medium with 500 nM of palbociclib. The IC₅₀ of sensitive MCF7 and T47D as well as derived palbociclib-resistant MCF7-PR and T47D-PR cell lines was evaluated by seeding 5000 tumor cells per well in 96-well plates and adding the following day increasing doses of Palbociclib. Proliferation was assessed after 4 days with CyQUANT NF Cell Proliferation (ThermoFisher Scientific) according to manufacturer's instructions. Fluorescence measurements were taken using FL800 Fluorescence plate reader with excitation at 485 nm and emission detection at 520 nm. Surviving fraction was calculated relative to untreated control cells using the formula: % Cell viability = (treated/untreated)*100 and IC⁵⁰ values were calculated with a non-linear regression model using GraphPad PRISM software.

These results showed that IC₅₀s of palbociclib-resistant cell lines are higher than the corresponding ones for sensitive cell lines (IC₅₀ MCF7 250nM, IC₅₀ MCF7-PR 2000nM; IC₅₀ T47D 50nM, IC₅₀ T47D-PR 1800nM) **(****Fig. 2A****).**

To uncover the potential role of FGFR4 in palbociclib-sensitive and palbociclib-resistant cell line models, the inventors used an FGFR4 siRNA-mediated knockdown. A panel of breast cancer cell line were transfected with 20 nmol/L siGENOME SMARTpool targeting FGFR4 (M-003134-02, FGFR4 siRNA) or Silencer^{®} Select Negative Control #1 (NT siRNA) (ThermoFisher Scientific) using Lipofectamine RNAiMax transfection reagent (Invitrogen) following the manufacturer's instructions. Cells were labeled after 6 days in culture with Hoechst 33342 (Invitrogen) and fluorescence was subsequently determined at the Gen5 Microplate Reader and Imager Software. Compared to cells transfected with the non-targeting (NT) siRNA, FGFR4 silencing significantly impaired the growth of palbociclib-resistant MCF7-PR and T47D-PR cells but not the growth of the sensitive parental MCF7 and T47D cells. In addition, FGFR4 silencing inhibited the growth of the intrinsically resistant ZR751 as well as the *FGFR4*-mutant cell line MDA-MB-453, which was used as a positive control (**Fig. 2B**). MDA-MB-453 cells predominantly express a mutated form of FGFR4, FGFR4 Y367C, which leads to ligand-independent activation of kinase activity and activation of signaling downstream of FGFR4 (Hagel et al., 2015).

Next, FGFR4-depletion was induced by means of stable shRNA expression and confirmed by Western blotting using standard procedures. Specifically, sensitive MCF7 and resistant MCFR7-PR and ZR751 were infected with two independent stable FGFR4 shRNAs (shFGFR4#1 and shFGFR4#2) and, as a control, a non-targeting scramble shRNA (shControl) **(****Fig.2C-2D****).** Response to palbociclib was evaluated by incubating the cells with increasing concentrations of palbociclib as described above. FGFR4 knockdown consistently reduced the IC₅₀s of both palbociclib-resistant cell lines, restoring sensitivity of MCF7-PR cells to palbociclib to the level of MCF7 parental cells (IC₅₀ MCF7-PR shControl 1553 nM, IC₅₀ MCF7-PR shFGFR4#1 576.4 nM, IC₅₀ MCF7-PR shFGFR4#2 449.3 nM; MCF7-PR shControl VS MCF7-PR shFGFR4#1 p<0.0001; MCF7-PR shControl VS MCF7-PR shFGFR4#2 p<0.0001) (**Fig. 2C**) and also restoring sensitivity of intrinsically resistant ZR751 (IC₅₀ ZR751 shControl 3803 nM, IC₅₀ ZR751 shFGFR4#1 80.23 nM, IC₅₀ ZR751 shFGFR4#2 124.7 nM; ZR751 shControl VS ZR751 shFGFR4#1 p=0.0009; ZR751 shControl VS ZR751 shFGFR4#2 p=0.0011) (**Fig. 2D**).

In order to validate FGFR4 as a driver of CDK4/6i resistance, the inventors developed ZR751 cells that overexpressed wild-type FGFR4 (FGFR4wt) or constitutively active FGFR4 (FGFR4 Y367C) (Roidl *et al.,* 2010). Control cells were infected with the backbone vector expressing red fluorescent protein (RFP). FGFR4 overexpression in ZR751 cells was confirmed (**Fig. 2E****, left**), and led to a decrease in the sensitivity to palbociclib in both FGFR4 overexpressing cell lines (IC₅₀ ZR751 RFP 353.8 nM, ZR751 FGFR4wt ~4.2e6, ZR751 FGFR4 Y367C ~1.2e16) (**Fig. 2E****, right**).

Collectively, these findings reveal an association between FGFR4 expression and resistance to CDK4/6i in breast cancer patients and breast cancer cell lines.

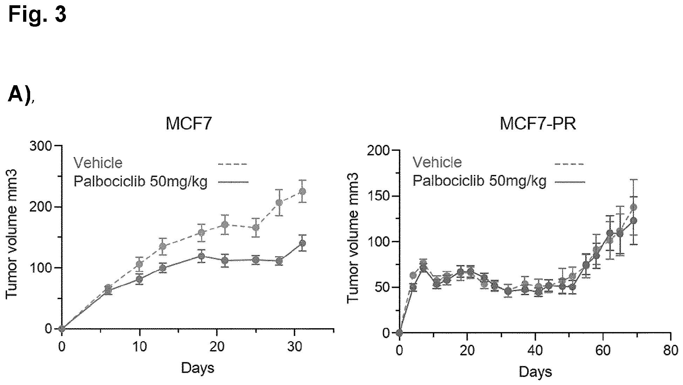

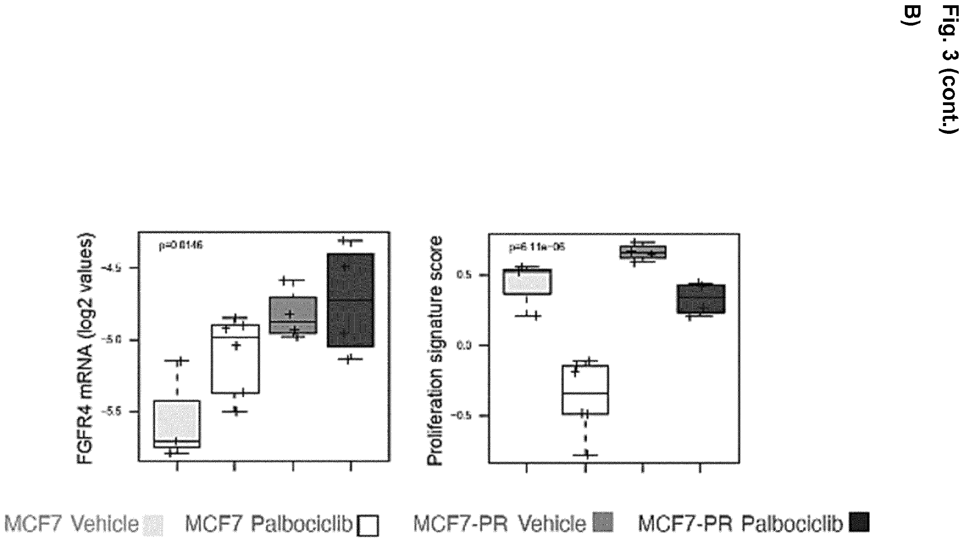

To test whether CDK4/6i resistance *in vivo* is dependent on FGFR4, the inventors developed an experimental model to test CDK4/6i systemic effects and explore experimental FGFR4 targeted treatments. 1×10⁶ MCF7 control and palbociclib-resistant (MCF7-PR) breast cancer cells were implanted in the mammary fat pad of female Balb/c nude mice and tumors were grown in the presence of supplemented oestrogens. Prior to inoculation, cells were grown as described above. Upon palpation (50 mm³), mice were randomized and treated orally with vehicle or palbociclib (50mg/kg) 5 days a week until the end of the experiment. During the length of the experiment, tumor volume was measured with a digital calliper twice per week. As previously reported, HR+ breast cancer experimental MCF7 model responded to CDK4/6i systemic treatment restraining tumor growth (vehicle VS palbociclib p=0.018) (**Fig**. **3A****, left panel**)**.** In contrast, palbociclib-resistant MCF7 derivatives (MFC7-PR) grew a lesion irrespectively of CDK4/6i treatment, confirming that palbociclib-resistance is maintained in vivo (vehicle VS palbociclib p=0.907) **(****Fig. 3A****, right panel).** After harvesting tumours, the inventors used the proliferation 60-gene nCounter panel to analyze FGFR4 gene expression and proliferation gene signature, and confirmed that palbociclib-resistant tumors expressed high levels of FGFR4 and showed increased proliferation score (**Fig. 3B**). These observations were confirmed by immunohistochemical (IHC) analysis of FGFR4. For that, FFPE-embedded tumors were cut at 4 µm and after standardized deparaffination, antigen retrieval was performed using heated citrate buffer, pH 6.0. Primary anti-FGFR4 antibody (4H2B10B2, Merck) was incubated at 1:500 dilution and detected using EnVision Flex system by Dako (Agilent) (**Fig. 3C**).

Next, the inventors studied the effect of genetically depleting FGFR4 in palbociclib-resistant MCF7 cells (MCF7-PR) *in vivo.* Control cells expressing scramble shRNA and FGFR4 knock-down cells expressing shRNA targeting FGFR4 (shFGFR4#1) were cultured and injected in mice following the same approach as that described above. When tumors reached approximately 50mm³ mice were randomized and treated orally with vehicle or palbociclib (50mg/kg) 5 days a week until the end of the experiment. Strikingly, FGFR4 depletion strongly prevented tumor growth *in vivo* in MCF7-PR cells (**Fig. 3D**), albeit the magnitude of the effect is amplified when mice are concomitantly treated with the CDK4/6i palbociclib, and suggesting synergistic effect in this latter case. These results indicate that FGFR4 targeting prevents tumour growth and restores CDK4/6i response.

### II. Identification of two monoclonal antibodies selective against FGFR4 to deliver a cytotoxic payload

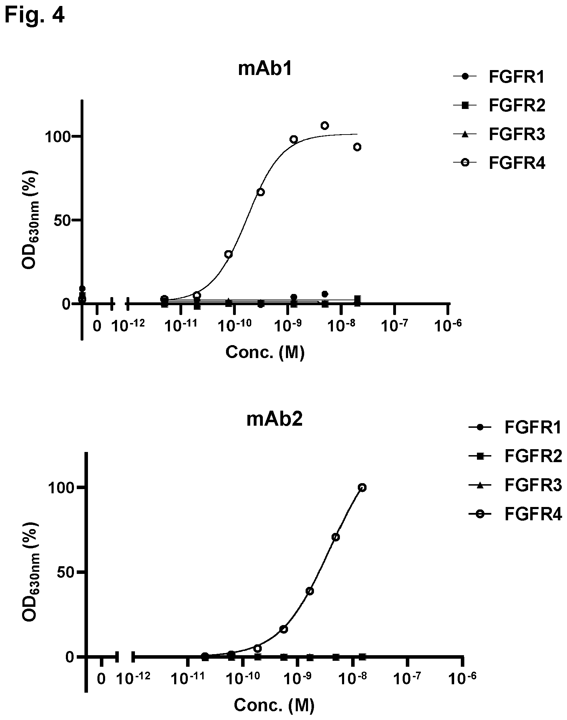

In a first step, we assessed several antibodies by their selectivity towards FGFR4. For that, a solid-phase binding assay was performed to assess the binding selectivity of mAb1 (CAT#: NS-028CN, Creative Biolabs) and mAb2 (CAT#: ARG53740, Arigo Laboratories) versus FGFR4 and the other members of the FGFR family (FGFR1, FGFR2, FGFR3). For that, MAXISORP flat bottom F96 Nunc plates were coated with recombinant His-tagged FGFRs 1-4 (Sino Biological) at 1µg/mL overnight at 4°C. After washing and blocking, increasing concentrations of mAb1 or mAb2 were added for 1h at room temperature (RT). Detection of antigen-bound antibody was carried out using an HRP-conjugated anti-mouse Fc secondary antibody (Dako) and revealed with TMB for a colorimetric reading at 630 nm. The results showed that mAb1 and mAb2_are selective binders against FGFR4, while they show negligible binding signal to FGFR1-3 (**Fig. 4**).

In an second stage, the inventors attempted to confirm the ability of these antibodies to deliver a toxic payload to cells expressing FGFR4. With that aim, specific anti-FGFR4 mAb1 and mAb2 antibodies were then used to deliver a commercial cytotoxic payload (Moradec, CAT#: AM-202-AE) to several cancer cell lines expressing FGFR4.

MDA-MB-453 breast cancer cell line was cultured according to provider recommendation (ATCC) and Huh7 hepatocellular cancer cell line (CLS) was cultured in DMEM supplemented with 10% FBS, 2 mM L-Glutamine, 1 mM sodium pyruvate and 1% penicillin-streptomycin (Gibco). MDA-MB-453 breast and Huh7 hepatocellular cancer cell lines were plated in triplicate and incubated with serial dilutions of control mouse IgG (hereinafter also referred as "isotype antibody", Absolute antibody CAT:Ab00102-1.1), mAb1 or mAb2 (at the indicated concentrations).

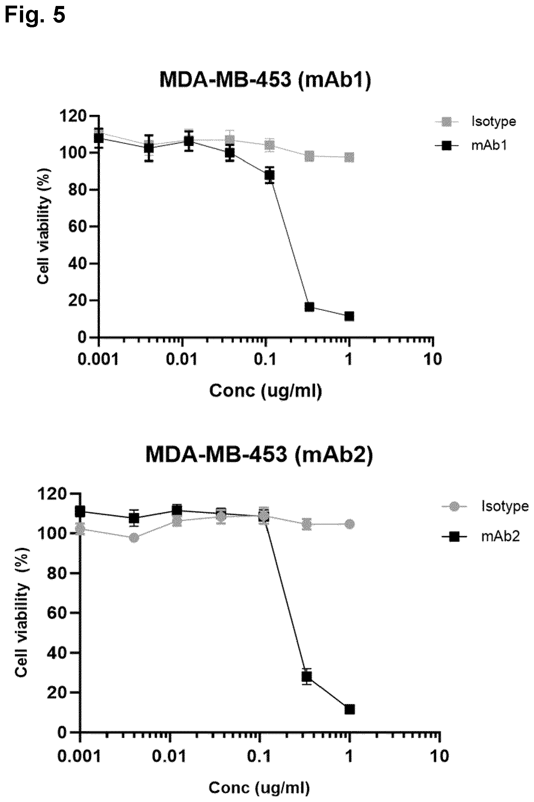

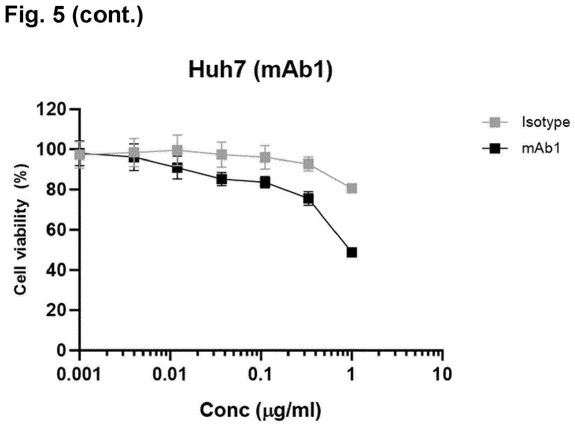

Subsequently, the cytotoxic payload (which includes an anti-Fc antibody conjugated to MMAE) was added at 6.6 nM and incubated for 6 days. At the end of the experiment, cell number was evaluated by adding CellTiter 96 Aqueous One Solution Reagent (Promega) following manufacturer's instructions. Finally, absorbance was recorded at 490 nm using TECAN Infinite MNano+ plate reader. These results showed dose-dependent effect of these anti-FGFR4 antibodies, while the isotype control had no effect. There results indicate that FGFR4 is essential for the effect and anti-FGFR4 specific antibodies can be used to deliver cytotoxic drugs to several cancer cells (Fig. 5).

### III. Preparation of an antibody-drug conjugate (ADC) of the invention suitable in the treatment of cancer

### ADC1 production

To a solution of mAb1 antibody (5 mg/mL) in phosphate-buffered saline, SuO-Val-Cit-PAB-MMAE (I) (13 eq, 5 mM in DMSO) was added and the mixture was reacted for 2h at 25 °C in an orbital shaker.. Average DAR was measured by high-resolution mass spectrometry, resulting in a DAR of 4.3 **(****Fig. 6****).**

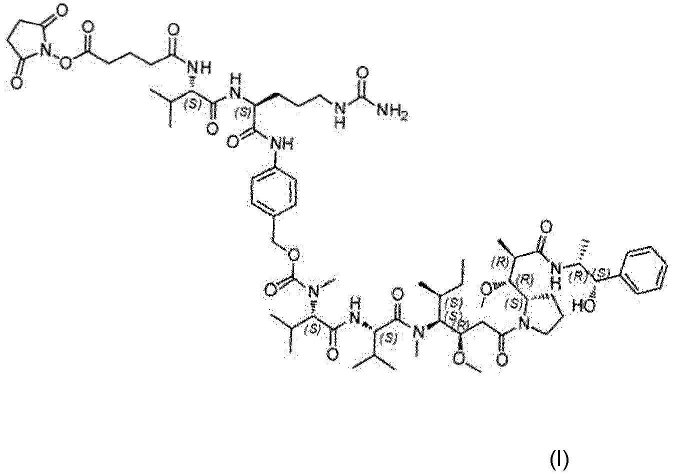

The reaction was quenched by the addition of 50 mM Tris·Cl, pH 7.4 and purified by repeated washing with phosphate-buffered saline using spin dialysis with 10 kDa MW cutoff membranes. Purity was assessed by SDS-PAGE and SEC-HPLC, using a MAbPac^{™} SEC-1 analytical column (ThermoFisher). For SEC-HPLC, 50 ul sample were injected at 1 mg/ml and run for 30 min at 0.15 ml/min in phosphate-buffered saline, pH 7.4. At 280 nm wavelength, the protein eluted as a single peak with no aggregates.

### ADC1 binding to FGFR4

Binding of ADC1 to FGFR4 was validated next by a solid-phase binding assay following the methodology described above. The results confirm that the antibody retained the ability to efficiently bind to FGFR4 once conjugated to the drug with similar affinity that the unconjugated antibody (IC₅₀ mAb1 0.93 nM, IC₅₀ ADC1 0.82 nM) **(****Fig. 7****).**

### Serum stability of ADC1

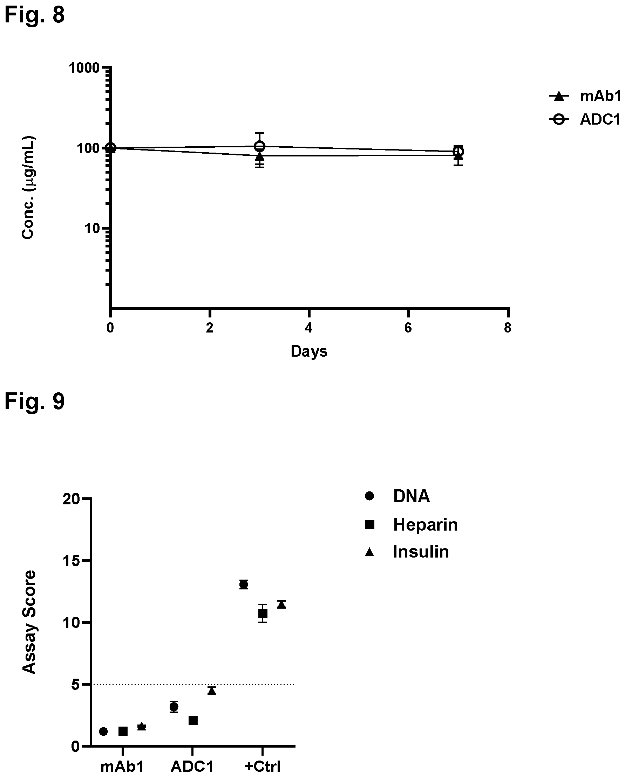

Mouse serum was spiked with naked mAb1 or ADC1 at 100 ug/mL and incubated at 37 °C for 7 days. Samples at day 3 and day 7 were quantified by FGFR4 solid-phase binding assay as described above. The results showed that the concentration of mAb1 and drug-conjugated ADC1 was nearly identical after 7 days, indicating that the ADC was stable and no significant differences with the naked antibody **(****Fig. 8****).**

### Non-specific binding of ADC1

The inventors evaluated the non-specific binding of ADC1 and mAb1 to DNA, heparin and insulin. For that, MAXISORP flat bottom F96 Nunc plates were coated overnight at 4 °C with DNA (10 µg/ml), heparin (10 µg/mL), insulin (5 µg/ml) in PBS pH 7.4. Wells were washed with water and blocked with 2% BSA in PBS for 1 hour at RT. Antibodies at 100 nM were added in triplicate to the wells and incubated for 1 h at room temperature. Plates were washed three times with water and non-specifically bound antibody was probed with an HRP-conjugated anti-human Fc secondary antibody (Dako). Assay scores were calculated as the ratio of the ELISA signal of the antibody at 100 nM to the signal of a well containing buffer instead of the primary antibody. Gantenerumab was used as positive control (Ctrl+; Jain et al. 2017). The results showed that the ADC had a score below 5, which is indicative of the specificity of the conjugate towards FGFR4 **(****Fig. 9****)**

### Pharmacokinetics (PK) study of ADC1 in mice

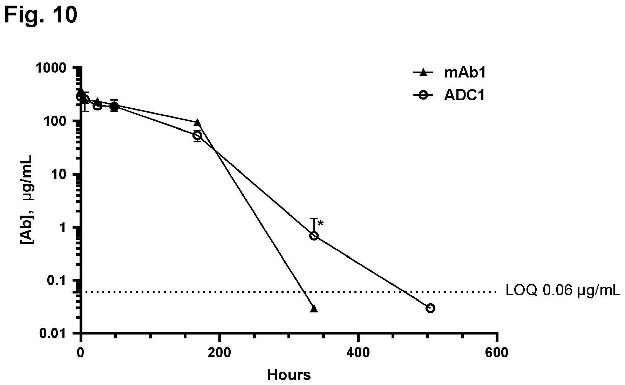

The pharmacokinetic properties of the ADC was evaluated by intravenously injecting Balb/c mice with ADC1 single 10 mg/kg dose and collecting plasma at 10 min, 6h, 24h, 48h, 7 days, 14 days and 21 days after injection using Heparin-Lithium blood collection capillary tubes. Blood samples were centrifuged at 2000 xg for 5 min at 4°C to obtain platelet-poor plasma and antibody levels were quantified using a FGFR4 solid-phase binding assay as described above. The corresponding concentration was determined using a non-linear regression 4-parameter standard calibration curve. Results showed that the antibody forming part of the conjugate (ADC1) exhibited a highly similar PK to the one shown by the antibody alone (mAb1) **(****Fig. 10****).**

### IV. Biological effect on cancer cells and cancer animal models

### Cytotoxicity assays performed with ADC1

Cytotoxic activity of ADC1 was evaluated in a panel of breast and hepatocellular cancer cell lines expressing FGFR4 (MDA-MB-453, ZR751, , T47D-PR, and Huh7) in addition to the FGFR4-negative breast cancer cell MDA-MB-231 (Protein Atlas, https://www.proteinatlas.org/ENSG00000160867-FGFR4/cell+line; Turunen et al, 2019). As a control, athe mouse IgG control antibody (used in previous sections) was conjugated to the same payload following the procedure described above as used (Isotype-ADC). Cell lines were cultured as detailed above and were seeded in triplicate in 96 well plates at a density of 12500 cells per well in 200 µl of cell culture medium. Following overnight incubation, medium was replaced with serial dilutions of ADC1s starting at 1 µg/ml. Medium only were also included as negative controls. At day 6, CellTiter 96 Aqueous One Solution Reagent (Promega,) was added to cells following manufacturer's instructions and absorbance was recorded at 490 nm using TECAN Infinite MNano+ plate reader. After subtraction of background absorbance, the effect of the ADC or isotype-ADC on cell proliferation was calculated for each replicate as follows: % Cell viability = (ADC treated/negative control)*100. FGFR4-targeting ADC, but not the non-targeting isotype-ADC, had cytotoxic activity at very low doses in cancer cell lines expressing FGFR4, independently of the cancer type, while it did not had any effect in the FGFR4-negative cell line **(****Fig.11****).**

### In vivo anti-tumoral activity of ADC1

Studies of the effects of ADC1 on tumoral growth were performed in female Balb/c nude mice of 12 weeks of age in the presence of supplemented oestrogens. All mice were subcutaneously injected in the back with 1×10⁶ ZR751 cells resuspended 1:1 in Matrigel:PBS1X. When tumors reached 100 mm³, mice were randomized to the various treatment groups: vehicle, isotype-ADC (described above) and ADC1. Treatments were administered intravenously once per week at a dose of 5 mg/kg or the corresponding volume in the case of vehicle-treated mice. Tumor volume was determined by caliper measurement until the end of the experiment. Treatment with FGFR4-specific ADC (ADC1) significantly reduced tumor growth compared to isotype-ADC or vehicle. Remarkably, ADC1 treatment resulted in tumoral regression at the end of the experiment (Isotype-ADC VS ADC1 p<0.0001; Vehicle VS ADC1 p<0.0001) **(****Fig. 12****).**

### References

Altschul et al. 1990 (J Mol Biol 215: 403-10),

Beenken, A., and Mohammadi, M. (2009). The FGF family: biology, pathophysiology and therapy. Nat. Rev. Drug Discov. 8, 235-253.

Brasó-Maristany, F., Griguolo, G., Pascual, T., Paré, L., Nuciforo, P., Llombart-Cussac, A., Bermejo, B., Oliveira, M., Morales, S., Martinez, N., et al. (2020). Phenotypic changes of HER2-positive breast cancer during and after dual HER2 blockade. Nat. Commun. 11, 385.

Cristofanilli, M., Turner, N.C., Bondarenko, I., Ro, J., Im, S.A., Masuda, N., Colleoni, M., DeMichele, A., Loi, S., Verma, S., et al. (2016). Fulvestrant plus palbociclib versus fulvestrant plus placebo for treatment of hormone-receptor-positive, HER2-negative metastatic breast cancer that progressed on previous endocrine therapy (PALOMA-3): final analysis of the multicentre, double-blind, phase 3 randomised controlled trial. Lancet Oncol. 17, 425-439.

Finn, R.S., Martin, M., Rugo, H.S., Jones, S., Im, S.-A., Gelmon, K., Harbeck, N., Lipatov, O.N., Walshe, J.M., Moulder, S., et al. (2016). Palbociclib and Letrozole in Advanced Breast Cancer. N. Engl. J. Med. 375, 1925-1936.

Goetz, M.P., Toi, M., Campone, M., Sohn, J., Paluch-Shimon, S., Huober, J., Park, I.H., Trédan, O., Chen, S.-C., Manso, L., et al. (2017). MONARCH 3: Abemaciclib As Initial Therapy for Advanced Breast Cancer. J. Clin. Oncol. 35, 3638-3646.

Hnasko, ELISA: Methods and Protocols (2015) Humana Press, chapter 6, p.61-69

Hortobagyi, G.N., Stemmer, S.M., Burris, H.A., Yap, Y.S., Sonke, G.S., Paluch-Shimon, S., Campone, M., Petrakova, K., Blackwell, K.L., Winer, E.P., etal. (2018). Updated results from MONALEESA-2, a phase III trial of first-line ribociclib plus letrozole versus placebo plus letrozole in hormone receptor-positive, HER2-negative advanced breast cancer. Ann. Oncol. 29, 1541-1547.

Im, S.-A., Lu, Y.-S., Bardia, A., Harbeck, N., Colleoni, M., Franke, F., Chow, L., Sohn, J., Lee, K.-S., Campos-Gomez, S., et al. (2019). Overall Survival with Ribociclib plus Endocrine

Therapy in Breast Cancer. N. Engl. J. Med. *381*, 307-316.

Jain T. et al., "Biophysical properties of the clinical-stage antibody landscape", PNAS, 2017, 114 (5), 944-949).

Johnston, S., Martin, M., Di Leo, A., Im, S.A., Awada, A., Forrester, T., Frenzel, M., Hardebeck, M.C., Cox, J., Barriga, S., et al. (2019). MONARCH 3 final PFS: a randomized study of abemaciclib as initial therapy for advanced breast cancer. Npj Breast Cancer 5, 1-8.

Modi S. et al. "Trastuzumab Deruxtecan in Previously Treated HER2-Low Advanced Breast Cancer". N Engl J Med 2022; 387:9-20

Prat A. et al. "Ribociclib plus letrozole versus chemotherapy for postmenopausal women with hormone receptor-positive, HER2-negative, luminal B breast cancer (CORALLEEN): an open-label, multicentre, randomised, phase 2 trial". Lancet Oncol. 2020 Jan;21(1):33-43.

Slamon, D.J., Neven, P., Chia, S., Fasching, P.A., De Laurentiis, M., Im, S.-A., Petrakova, K., Bianchi, G.V., Esteva, F.J., Martin, M., et al. (2018). Phase III Randomized Study of Ribociclib and Fulvestrant in Hormone Receptor-Positive, Human Epidermal Growth Factor Receptor 2-Negative Advanced Breast Cancer: MONALEESA-3. J. Clin. Oncol. 36, JCO.2018.78.990.

Sledge, G.W., Toi, M., Neven, P., Sohn, J., Inoue, K., Pivot, X., Burdaeva, O., Okera, M., Masuda, N., Kaufman, P.A., et al. (2017). MONARCH 2: Abemaciclib in Combination With Fulvestrant in Women With HR+/HER2- Advanced Breast Cancer Who Had Progressed While Receiving Endocrine Therapy. J. Clin. Oncol. 35, 2875-2884.

Sledge, G.W., Toi, M., Neven, P., Sohn, J., Inoue, K., Pivot, X., Burdaeva, O., Okera, M., Masuda, N., Kaufman, P.A., et al. (2020). The Effect of Abemaciclib Plus Fulvestrant on Overall Survival in Hormone Receptor-Positive, ERBB2-Negative Breast Cancer That Progressed on Endocrine Therapy - MONARCH 2: A Randomized Clinical Trial. JAMA Oncol. 6, 116-124.

Tripathy, D., Im, S.A., Colleoni, M., Franke, F., Bardia, A., Harbeck, N., Hurvitz, S.A., Chow, L., Sohn, J., Lee, K.S., et al. (2018). Ribociclib plus endocrine therapy for premenopausal women with hormone-receptor-positive, advanced breast cancer (MONALEESA-7): a randomised phase 3 trial. Lancet Oncol. 19, 904-915.

Turner, N., and Grose, R. (2010). Fibroblast growth factor signalling: from development to cancer. Nat. Rev. Cancer 10, 116-129.

Turner, N.C., Slamon, D.J., Ro, J., Bondarenko, I., Im, S.-A., Masuda, N., Colleoni, M., DeMichele, A., Loi, S., Verma, S., et al. (2018). Overall Survival with Palbociclib and Fulvestrant in Advanced Breast Cancer. N. Engl. J. Med. 379, 1926-1936.

Turunen SP, von Nandelstadh P, Ohman T, Gucciardo E, Seashore-Ludlow B, Martins B, Rantanen V, Li H, Höpfner K, Ostling P, Varjosalo M, Lehti K. F (2019). FGFR4 phosphorylates MST1 to confer breast cancer cells resistance to MST1/2-dependent apoptosis. Cell Death Differ 26, 2577-2593

Ruggeri B. et al., "The novel FGFR4-selective inhibitor INCB062079 is efficacious in models of hepatocellular carcinoma harboring FGF19 amplification [abstract]. Cancer Res. 2017;77:1234.

### Clauses

For reasons of completeness, various aspects of the invention are set out in the following numbered clauses:
**Clause 1.** A method for determining the response of a subject already diagnosed of having cancer to a medical regimen comprising one or more CDK4/6 inhibitors, the method comprising the steps of
   (a) determining the level of expression of FGFR4 in an isolated test sample of the subject, and
   (b) comparing with a reference value,
   wherein if FGFR4 is overexpressed, then this is indicative that there will be a reduced or absence of response to the one or more CDK4/6 inhibitors.
**Clause 2.** The method of clause 1, wherein the level of expression of FGFR4 is determined prior to start the medical regimen.
**Clause 3.** The method of clause 2, wherein the subject is the first time that is going to be administered with the medical regimen comprising the one or more CDK4/6 inhibitors.
**Clause 4.** The method of any one of the preceding clauses, wherein the subject has already been treated with one or more CDK4/6 inhibitors prior to determining the level of expression of FGFR4.
**Clause 5.** The method of any of the clauses 1-2, wherein the level of expression of FGFR4 is determined during the medical regimen.
**Clause 6.** The method of any one of the preceding clauses, wherein the one or more CDK4/6 inhibitors are selected from palbociclib, ribociclib or abemaciclib; particularly the medical regimen comprises administering palbociclib.
**Clause 7.** The method of any one of the preceding clauses, wherein the medical regimen further comprises an endocrine therapy.
**Clause 8.** The method of any one of the preceding clauses, wherein determining the level of expression of FGFR4 comprises determining the amount of mRNA FGFR4.
**Clause 9.** The method of any one of the preceding clauses, wherein the level of expression of FGFR4 is determined by PCR.
**Clause 10.** The method according to any of the preceding clauses, wherein the level of expression is determined at the protein level.
**Clause 11.** The method according to clause 10, wherein the level of expression of protein is determined using an antibody or a fragment thereof able to bind to the protein.
**Clause 12.** The method according to clause 11, wherein said antibody or fragment thereof forms part of a kit.
**Clause 13.** The method of any one of the preceding clauses, wherein the test sample is selected from a biological fluid sample (such as serum, blood, plasma), a tissue sample (such as a tissue homogenate) or a cell lysate.
**Clause 14.** Use of FGFR4 as a marker for predicting or monitoring the response of a subject to a medical regimen comprising one or more CDK4/6 inhibitors.
**Clause 15.** Use of means for determining the level of expression of FGFR4 in a method as defined in any of the clauses 1-14.
**Clause 16.** The method of any one of the clauses 1-13 or the use according to any of the clauses 14-15, wherein the subject suffers a breast cancer, particularly a HR(+)-breast cancer; particularly HR(+)/HER2-negative breast cancer.
**Clause 17.** A CDK4/6 inhibitor for use in a method of treating cancer in a subject, wherein the method comprises:
   (i) determining the expression level of FGFR4 in an isolated test sample of the subject,
   (ii) comparing with a reference value, and
   (ii) administering the CDK4/6 inhibitor to the subject if the level of expression of FGFR4 in the test sample is equal or lower than the one in the reference value.
**Clause 18.** An anti-FGFR4 antibody drug conjugate (ADC), wherein the anti-FGFR4 antibody:
   - directly binds to FGFR4, and
   - is not specific of FGFR1, FGFR2, and FGFR3.
**Clause 19.** The ADC according to clause 18, wherein the antibody directly binds to FGFR4.
**Clause 20.** The ADC according to any of the preceding clauses 18-19, wherein the drug binds to the antibody through a cleavable linker.
**Clause 21.** The ADC according to any one of the preceding clauses, wherein the cleavable linker is a peptidyl linker cleavable by an intracellular protease, particularly a Val-Cit linker or a Phe-Lys linker.
**Clause 22.** The ADC according to any one of the preceding clauses, wherein the drug binds, through the cleavable linker, to a Lys residue of the antibody.
**Clause 23.** The ADC according to any one of the preceding clauses, wherein the antibody is a human or humanized antibody.
**Clause 24.** The ADC according to any one of the preceding clauses, wherein the drug is an auristatin, particularly monomethyl auristatin E.
**Clause 25.** The ADC according to clause 24, wherein the drug-antibody ratio is from 2 to 6, particularly 4.
**Clause 26.** A pharmaceutical composition comprising the antibody drug conjugate of any one of claims 18-25 and a pharmaceutically acceptable carrier.
**Clause 27.** The pharmaceutical composition of clause 26, wherein said composition is a lyophilised composition.
**Clause 28.** The anti-FGFR4 ADC according to any one of the preceding clauses 18-25 or the pharmaceutical composition according to any one of the clauses 26-27, for use in the treatment of a subject suffering from a cancer expressing FGFR4.
**Clause 29.** The ADC for use according to clause 28, wherein the subject suffers from a cancer overexpression FGFR4.
**Clause 30.** The ADC for use according to any one of the preceding clauses 28-29, wherein the cancer is selected from oral squamous cell carcinoma, head and neck cancer, esophageal cancer, gastric cancer, ovarian cancer, cervical cancer, lung cancer, breast cancer, renal cancer, prostate cancer, sarcoma, melanoma, leukemia, lymphoma, kidney cancer, duodenum cancer, small intestine cancer, liver cancer, pancreatic cancer, bladder cancer, rhabdomyosarcoma, or cholangiocarcinoma; particularly the cancer is breast cancer, liver cancer, gastric cancer or colorectal cancer.
**Clause 31.** The ADC for use according to any one of the preceding clauses 28-30, wherein the subject is further resistant to a medical regimen comprising one or more CDK4/6 inhibitors.
**Clause 32.** The ADC for use according to any one of the preceding clauses 28-31, wherein the ADC or composition is administered in combination with one or more therapeutic agents selected from the group consisting of: anti-cancer agents, anti-allergic agents, anti-nausea agents (or anti-emetics), pain relievers, cytoprotective agents, and combinations thereof.
**Clause 33.** A FGFR4 inhibitor for use in reducing the resistance to CDK4/6 inhibitor(s) in a subject suffering from cancer.
**Clause 34.** A FGFR4 inhibitor for use in treating cancer in a subject that is resistant to CDK4/6 inhibitor.
**Clause 35.** The FGFR4 inhibitor for use according to any one of the clauses 33-34, which is selected from a small molecule, an antibody anti-FGFR4 or an antibody anti-FGFR4-drug conjugate.
**Clause 36.** The FGFR4 inhibitor for use according to clause 35, which is an antibody anti-FGFR4.
**Clause 37.** The FGFR4 inhibitor for use according to clause 36, wherein the antibody does not bind to FGFR1, FGFR2 neither FGFR3.
**Clause 38.** The FGFR4 inhibitor for use according to any one of the clauses 33-37, which is an antibody anti-FGFR4-drug conjugate as defined in any of the clauses 19 to 25.
**Clause 39.** The FGFR4 inhibitor for use according to any one of the clauses 33-38, wherein the subject suffers from breast cancer.
**Clause 40.** The FGFR4 inhibitor for use according to any one of the clauses 33-39, which is administered in combination with one or more anti-cancer agents, anti-allergic agents, anti-nausea agents (or anti-emetics), pain relievers, cytoprotective agents, and combinations thereof.
**Clause 41.** A method for deciding or recommending initiating the administration of an ADC as defined herein, for a subject which suffers from, or is suspicious of having, cancer, the method comprising the step of determining the level of expression of FGFR4; and, if it is confirmed the expression of FGFR4, then it is recommended the administration of the ADC of the invention.

## Claims

1. An anti-FGFR4 antibody drug conjugate (ADC) for use in the treatment or prevention of a cancer expressing FGFR4, wherein the anti-FGFR4 antibody:
- directly binds to FGFR4, and
- is not specific of FGFR1, FGFR2, and FGFR3.

2. The ADC for use according to claim 1, wherein the drug binds to the antibody through a linker which is a cleavable linker.

3. The ADC for use according to any one of the preceding claims, wherein the cleavable linker is a peptidyl linker cleavable by an intracellular protease, particularly a valine-citrulline linker or a Phe-Lys linker; particularly, the linker is a valine-citrulline-p-aminocarbamate (VC-PABC).

4. The ADC for use according to any one of the preceding claims, wherein the drug binds, through the cleavable linker, to one or more Lys residues of the antibody.

5. The ADC for use according to any one of the preceding claims, wherein the drug is an auristatin, particularly monomethyl auristatin E.

6. The ADC for use according to claim 5, wherein the drug-antibody ratio is from 1 to 10, particularly from 2 to 6, particularly from 3 to 5.

7. The ADC for use according to any one of the preceding claims, which is administered in the form of a pharmaceutical composition, together with one or more pharmaceutically acceptable carriers and/or excipients.

8. The ADC for use according to any one of the preceding claims, wherein the subject suffers from a cancer correlating with overexpression of FGFR4.

9. The ADC for use according to any one of the preceding claims, wherein the subject is further resistant to a medical regimen comprising one or more CDK4/6 inhibitors.

10. A FGFR4 inhibitor for use in reducing the resistance to CDK4/6 inhibitor(s) in a subject suffering from cancer.

11. A FGFR4 inhibitor for use in a method of treating or preventing cancer in a subject that is resistant to CDK4/6 inhibitor.

12. The FGFR4 inhibitor for use according to claim 11, wherein the FGFR4 inhibitor does not bind to FGFR1, FGFR2 neither FGFR3.

13. The FGFR4 inhibitor for use according to any one of the claims 11-12, which is selected from a small molecule, an antibody anti-FGFR4 or an antibody anti-FGFR4-drug conjugate; particularly the FGFR4 inhibitor is an antibody anti-FGFR4-drug conjugate.

14. The FGFR4 inhibitor for use according to any one of the claims 11-13, which is an antibody anti-FGFR4-drug conjugate as defined in any of the claims 1 to 6.

15. The FGFR4 inhibitor for use according to any one of the claims 11-14, wherein the subject suffers from a cancer selected from oral squamous cell carcinoma, head and neck cancer, esophageal cancer, gastric cancer, ovarian cancer, cervical cancer, lung cancer, breast cancer, renal cancer, prostate cancer, sarcoma, melanoma, leukemia, lymphoma, kidney cancer, duodenum cancer, small intestine cancer, liver cancer, pancreatic cancer, bladder cancer, rhabdomyosarcoma, or cholangiocarcinoma; particularly the cancer is breast cancer, liver cancer, gastric cancer or colorectal cancer; more particularly the cancer is breast cancer.
